# EUROPEAN PATENT SPECIFICATION

(11) **EP 4 453 243 B1**
(45) Date of publication and mention of the grant of the patent: **11.02.2026**
(21) Application number: 22857058.6
(22) Date of filing: 16.12.2022
(51) Int. Cl.: C12Q 1/6806, C12Q 1/6874

(54) **PERIODATE COMPOSITIONS AND METHODS FOR CHEMICAL CLEAVAGE OF SURFACE-BOUND POLYNUCLEOTIDES**
PERIODATZUSAMMENSETZUNGEN UND VERFAHREN ZUR CHEMISCHEN SPALTUNG VON OBERFLÄCHENGEBUNDENEN POLYNUKLEOTIDEN
COMPOSITIONS DE PERIODATE ET MÉTHODES DE CLIVAGE CHIMIQUE DE POLYNUCLÉOTIDES LIÉS À LA SURFACE

(30) Priority: 20.12.2021 US 202163291862 P
(43) Date of publication of application: 30.10.2024
(73) Proprietor: ILLUMINA, INC., San Diego, CA 92122 (US)
(72) Inventor: MILLER, Oliver, Cambridge Cambridgeshire CB21 6DF (GB); MARAFINI, Pietro, Cambridge Cambridgeshire CB21 6DF (GB)
(74) Representative: Murgitroyd & Company
(86) International application number: PCT/US2022/081764
(87) International publication number: WO 2023/122491

(56) References cited:
- EP-A2- 2 021 415
- US-A1- 2005 164 207
- US-A1- 2019 352 327

## Description

### Field

Embodiments of the present disclosure relate to compositions and methods of chemical linearization of double-stranded polynucleotides for sequencing-by-synthesis (SBS).

### REFERENCE TO SEQUENCE LISTING

The present application is being filed along with a Sequence Listing in electronic format. The Sequence Listing is provided as a .xml file entitled Sequence_listing_ILLINC_691WO.xml created December 16, 2022, which is 18.0 KB in size. The information in the electronic format of the Sequence Listing is incorporated herein by reference in its entirety.

### BACKGROUND

Various nucleic acid sequencing methods are known in the art. U.S. Patent No. 5,302,509 describes a method for sequencing a polynucleotide template that involves performing multiple extension reactions using a DNA polymerase or DNA ligase to successively incorporate labelled polynucleotides complementary to a template strand. In such a SBS reaction, a new polynucleotide strand based-paired to the template strand is built up in the 5' to 3' direction by successive incorporation of individual nucleotides complementary to the template strand. The substrate nucleoside triphosphates used in the sequencing reaction are labelled at the 3' position with different 3' labels, permitting determination of the identity of the incorporated nucleotide as successive nucleotides are added.

In order to maximize the throughput of nucleic acid sequencing reactions it is advantageous to be able to sequence multiple template molecules in parallel. Parallel processing of multiple templates can be achieved with the use of nucleic acid array technology. These arrays typically consist of a high-density matrix of polynucleotides immobilized onto a solid support material.

Various methods for fabrication of arrays of immobilized nucleic assays have been described in the art. WO 98/44151 and WO 00/18957 both describe methods of nucleic acid amplification which allow amplification products to be immobilized on a solid support in order to form arrays comprised of clusters or "colonies" formed from a plurality of identical immobilized polynucleotide strands and a plurality of identical immobilized complementary strands. Arrays of this type are referred to herein as "clustered arrays." The nucleic acid molecules present in DNA colonies on the clustered arrays prepared according to these methods can provide templates for sequencing reactions, for example as described in WO 98/44152. The products of solid-phase amplification reactions such as those described in WO 98/44151 and WO 00/18957 are so-called "bridged" structures formed by annealing of pairs of immobilized polynucleotide strands and immobilized complementary strands, both strands being attached to the solid support at the 5' end. In order to provide more suitable templates for nucleic acid sequencing, it is preferred to remove substantially all or at least a portion of one of the immobilized strands in the "bridged" structure in order to generate a template which is at least partially single-stranded. The portion of the template which is single-stranded will thus be available for hybridization to a sequencing primer. The process of removing all or a portion of one immobilized strand in a "bridged" double-stranded nucleic acid structure is referred to as "linearization." There are various ways for linearization, including but not limited to enzymatic cleavage, photo-chemical cleavage, or chemical cleavage. Non-limiting examples of linearization methods are disclosed in PCT Publication No. WO 2007/010251 and U.S. Patent Publication No. 2009/0088327, and in U.S. Patent Publication No. 2009/01181 28.

EP 2 021 415 A2 discloses a cleaving method of products containing diol linkages wherein the method uses periodate.

US 2005/164207 A1 relates to a cleavage of Oligonucleotides from array using 1, 2-diol cleavable linker wherein the cleavage is carried out in a periodate solution.

US 2019/352327 A1 relates to methods of preparation of templates for polynucleotide sequencing. The diol linker is cleaved by treatment with a "cleaving agent", which can be any substance which promotes cleavage of the diol.

Enzymatic methods are known to facilitate efficient site-specific cleavage of oligonucleotides or polynucleotides to linearize double stranded DNA clusters and to deprotect surface-bound primers. Currently, enzymes have been extensively used in both of these types of reactions in various sequencing applications. However, there are certain issues with the enzymatic approaches, including enzyme stability, costs of enzyme production, specific storage and handling requirements, variations in enzyme activity, and high background intensity in sequencing reading. Therefore, there exists a need to develop alternative linearization and deprotection methods for effective DNA sequencing. However, there are many limitations on the reaction types that can be applied to linearization steps in this context, as the reagents, conditions, and byproducts (a) must be compatible with up- and downstream reactions, including oligonucleotide hybridization and denature, primer PCR extension, and DNA synthesis, (b) must display good stability under acidic, basic, and oxidative conditions, (c) must effect a rapid and clean chemical reaction, and (d) must not interfere with nucleotide detection methods. The present disclosure describes compositions for chemical cleavage of double stranded DNA that is an effective alternative that meets the requirements described above.

### SUMMARY

One aspect of the present disclosure relates to a composition comprising a periodate salt and an ionic liquid additive, wherein the ionic liquid additive is a substituted imidazolium salt, wherein the periodate is dissolved in an aqueous solution, and the periodate salt does not form a precipitate in the aqueous solution.

In some embodiments of the composition described herein, the concentration of the periodate salt in the composition is composition is from about 0.1 mM to about 300 mM, from about 0.5 mM to about 200 mM, from about 1 mM to about 150 mM, from about 2 mM to about 100 mM, or from about 5 mM to about 50 mM. In one embodiment, the concentration of the periodate salt in the composition is about 10 mM. In another embodiment, the concentration of the periodate salt in the composition is about 25 mM.

In other embodiments, the substituted imidazolium salt comprises or is 1-benzyl-3-methylimidazolium chloride ([Bzmim]Cl). In some embodiments, the molar ratio of 15-crown-5 to the periodate salt in the composition is from about 1:1 to about 50:1, from about 5:1 to about 25:1, or about 10:1. In some embodiments, the molar ratio of [Bzmim]Cl to the periodate salt in the composition is from about 1:1 to about 100:1, from about 10:1 to about 50:1, or about 30:1.

In some embodiments of the composition described herein, the composition further comprises one or more inorganic salts. For example, the inorganic salt may comprise one or more sodium salts, or one or more magnesium salts, or a combination thereof. In further embodiments, the inorganic salt may comprise sodium citrate, sodium acetate, magnesium sulfate, or combinations thereof.

In some embodiments of the composition described herein, the pH of the composition is from about 4.0 to about 8.0, about 5.0 to about 7.5, or about 6.0. In further embodiments, no precipitate of the periodate salt is formed after the composition is subject to at least 5, 6, 7, 8, or 9 freeze/thaw cycles.

In any embodiments of the composition described herein, the periodate salt comprises or is sodium periodate.

Another aspect of the present disclosure relates to a method of linearizing a plurality of immobilized double-stranded polynucleotides, comprising:
contacting a composition containing a periodate salt as described herein with a solid support comprising the plurality of immobilized double-stranded polynucleotides, each double-stranded polynucleotide comprises a first strand and a second strand, wherein the first strand and the second strand are immobilized to the solid support at their 5' ends, wherein each second strand comprises a cleavage site; and
chemically cleaving one or more second strands at the cleavage site with the periodate salt, and generating one or more cleaved second nucleic acids.

In some embodiments of the linearization method described herein, the method further comprises removing the cleaved second nucleic acids from the solid support. In some embodiments, the cleavage site of each second strand comprises one or more diol linkers. In some embodiments, the diol linker comprises a structure of Formula (I): 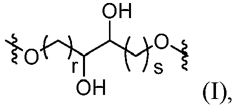 wherein r is 2, 3, 4, 5, or 6; and s is 2, 3, 4, 5, or 6. In one embodiment, the diol linker comprises a structure of Formula (Ia): 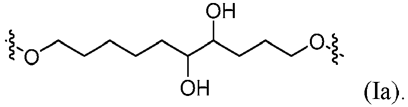 In further embodiments, the second strand polynucleotide comprises a P17 sequence.

Another aspect of the present disclosure relates to a method of sequencing polynucleotides, comprising:
contacting a palladium catalyst with a solid support comprising a plurality of immobilized double-stranded polynucleotides, each double-stranded polynucleotide comprises a first strand and a second strand, wherein the first strand and the second strand are immobilized to the solid support at their 5' ends, wherein each first strand comprises a first cleavage site comprising a vinyl moiety, and wherein each second strand comprises a second cleavage site comprising one or more diol linkers;
cleaving one or more first strands at the first cleavage site with the palladium catalyst, and generating one or more cleaved first nucleic acids and cleaved immobilized first strands;
removing the cleaved first nucleic acids from the solid support;
sequencing the immobilized second strands;
resynthesizing derivative first strands that are complementary to the second strands;
contacting a composition containing a periodate salt as described herein with one or more second strands to cleave the second strands at the second cleavage site, and generating one or more cleaved second nucleic acids and cleaved immobilized second strands;
removing the one or more cleaved second nucleic acids from the solid support; and
sequencing the immobilized derivative first strands.

In some embodiments of the sequencing method described herein, the first cleavage site comprises a modified nucleotide comprising a structure of Formula (II): 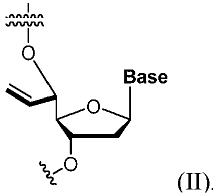 , wherein Base is adenine, 7-deazaademine, guanine, 7-deazaguanine, cytosine, thymine, or uracil, or a derivative thereof. In further embodiments, each first strand is extended from a first extension primer immobilized to the solid support, and wherein the first extension primer comprises a P15 sequence. In some embodiments, the diol linker comprises a structure of Formula (I): 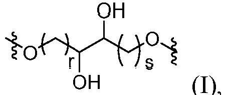 wherein r is 2, 3, 4, 5, or 6; and s is 2, 3, 4, 5, or 6. In one embodiment, the diol linker comprises a structure of Formula (Ia): 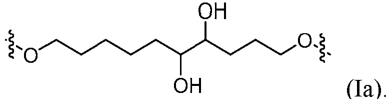 In further embodiments, each second strand is extended from a second extension primer immobilized to the solid support, and wherein the second extension primer comprises a P17 sequence.

A further aspect of the present disclosure relates to a kit for chemical linearization of double stranded polynucleotides, comprising a periodate salt and an ionic liquid additive, wherein the ionic liquid additive is a substituted imidazolium salt, wherein the periodate salt and the ionic liquid additive are in a lyophilized form.

In some such embodiments, the crown ether comprises or is 15-crown-5. In other embodiments, the substituted imidazolium salt comprises or is [Bzmim]Cl. In some embodiments, the molar ratio of 15-crown-5 to the periodate salt is from about 1:1 to about 50:1, from about 5:1 to about 25:1, or about 10:1. In some embodiments, the molar ratio of [Bzmim]Cl to the periodate salt is from about 1:1 to about 100:1, from about 10:1 to about 50:1, or about 30:1. In some embodiments of the kit described herein, the kit further comprises one or more inorganic salts. For example, the inorganic salt may comprise one or more sodium salts, or one or more magnesium salts, or a combination thereof. In further embodiments, the inorganic salt may comprise sodium citrate, sodium acetate, magnesium sulfate, or combinations thereof.

### BRIEF DESCRIPTION OF THE DRAWINGS

**FIG. 1** illustrates an embodiment of a workflow of the Illumina's Sequencing-by-Synthesis (SBS) chemistry using chemical linearization.
**FIG. 2** is a bar chart of percent linearization of double-stranded polynucleotides in a composition comprising sodium periodate when different ionic liquid additive is added.
**FIG. 3** is a bar chart of percent linearization of double-stranded polynucleotides in a composition comprising sodium periodate at various conditions when different ionic liquid additive is added.
**FIG. 4A** is a line chart illustrating the linearization rate as a function of sodium periodate concentration when 30.4 molar equivalent of [Bzmim]Cl is added to a composition comprising sodium periodate.
**FIG. 4B** is a line chart illustrating the linearization rate as a function of sodium periodate concentration where no ionic liquid additive is present.
**FIG. 5** is a bar chart comparing the percent of initial rate of diol cleavage of double-stranded polynucleotides using a freshly prepared sodium periodate composition compared to the same composition that is aged in air at 60° for 10 days.

### DETAILED DESCRIPTION

The invention is defined in the appended claims.

Non-enzymatic chemical linearization strategies are an attractive alternative for cleaving the bridged double-stranded polynucleotide structures ahead of each sequencing read. In particular, chemicals can often be stored for prolonged periods at room temperature and are relatively inexpensive compared to enzymes. Furthermore, chemical compositions may further be shipped and/or stored in a lyophilized form and be reconstituted into an aqueous solution prior to use. If required, one or both strands of the double-stranded nucleic acid molecule may include one or more non-nucleotide chemical moieties and/or non-natural nucleotides and/or non- natural backbone linkages to permit a chemical cleavage reaction at a specific cleavage site, preferably a pre-determined cleavage site.

Diol linker units based on phosphoramidite chemistry suitable for incorporation into polynucleotide chains are commercially available from Fidelity Systems, Inc. (Gaithersburg, MD, USA). One or more diol units may be incorporated into a polynucleotide using standard methods for automated chemical DNA synthesis. In order to position the diol linker at an optimum distance from the solid support one or more spacer molecules may be included between the diol linker and the site of attachment to the solid support. The spacer molecule may be a non-nucleotide chemical moiety. Suitable spacer units based on phosphoramidite chemistry for use in conjunction with diol linkers are also supplied by Fidelity Systems, Inc. The diol linker is cleaved by treatment with a "cleaving agent", which can be any substance which promotes cleavage of the diol. The preferred cleaving agent is periodate, preferably aqueous sodium periodate (NaIO₄). Following treatment with the cleaving agent (e.g., periodate) to cleave the diol, the cleaved product may be treated with a "capping agent" in order to neutralize reactive species generated in the cleavage reaction. Suitable capping agents for this purpose include amines, such as ethanolamine. Advantageously, the capping agent (e.g., ethanolamine) may be included in a mixture with the cleaving agent (e.g., periodate) so that reactive species are capped as soon as they are formed. In one non-limiting embodiment, one strand of the double-stranded nucleic acid molecule may include a diol linkage which permits cleavage by treatment with periodate (e.g., sodium periodate). The diol linkage may be positioned at a cleavage site, the precise location of which may be selected by the user. It will be appreciated that more than one diol could be included at the cleavage site.

The combination of a diol linkage and a periodate salt to achieve cleavage of one strand of a double-stranded nucleic acid molecule is preferred for linearization of nucleic acid molecules on solid supported polyacrylamide hydrogels because treatment with periodate is compatible with nucleic acid integrity and with the chemistry of the hydrogel surface. However, sodium periodate aqueous solution tends to form a precipitate after several freeze/thaw cycles. The formation of the precipitate may result in decreased linearization rate or efficiency.

Embodiments of the present disclosure relates to an improved periodate composition comprising an ionic liquid additive to substantially reduce or prevent the formation of precipitate after repeated freeze/thaw cycles. The periodate composition is stable in the air for an extended period of time and also provide accelerated linearization rate compared to the composition without the ionic liquid additive. Furthermore, methods of chemical linearization of double-stranded polynucleotides and the subsequent sequencing application are also described herein. The periodate composition described herein may also improve the chemical linearization rate of the double-stranded polynucleotides by at least 100%, 200%, or 300% as compared to the chemical linearization rate using the same periodate composition without the ionic liquid additive.

### Definitions

The section headings used herein are for organizational purposes only and are not to be construed as limiting the subject matter described.

Unless defined otherwise, all technical and scientific terms used herein have the same meaning as is commonly understood by one of ordinary skill in the art. The use of the term "including" as well as other forms, such as "include", "includes," and "included," is not limiting. The use of the term "having" as well as other forms, such as "have", "has," and "had," is not limiting. As used in this specification, whether in a transitional phrase or in the body of the claim, the terms "comprise(s)" and "comprising" are to be interpreted as having an open-ended meaning. That is, the above terms are to be interpreted synonymously with the phrases "having at least" or "including at least." For example, when used in the context of a process, the term "comprising" means that the process includes at least the recited steps, but may include additional steps. When used in the context of a compound, composition, or device, the term "comprising" means that the compound, composition, or device includes at least the recited features or components, but may also include additional features or components.

As used herein, the term "covalently attached" or "covalently bonded" refers to the forming of a chemical bonding that is characterized by the sharing of pairs of electrons between atoms. For example, a covalently attached polymer coating refers to a polymer coating that forms chemical bonds with a functionalized surface of a substrate, as compared to attachment to the surface via other means, for example, adhesion or electrostatic interaction.

As used herein, the term "extension primer" refers to an oligonucleotide or polynucleotide immobilized on a solid support, where the oligonucleotide or polynucleotide is capable of specifically binding to a sequence of a target single strand nucleic acid molecule. After a hybridization process, the oligonucleotide or polynucleotide is extended to comprise sequence that is complimentary to the target nucleic acid molecule. In some instances, the term "extension primer" is used interchangeably with "amplification primer." The extension primer described herein may include P5/P7, or P15/P17 primers. The P5 and P7 primers are used on the surface of commercial flow cells sold by Illumina Inc. for sequencing on the Specific examples of suitable primers include P5 and/or P7 primers, which are used on the surface of commercial flow cells sold by Illumina, Inc., for sequencing on HISEQ^{™}, HISEQX^{™}, MISEQ^{™}, MISEQDX^{™}, MINISEQ^{™}, NEXTSEQ^{™}, NEXTSEQDX^{™}, NOVASEQ^{™}, GENOME ANALYZER^{™}, ISEQ^{™}, and other instrument platforms. The primer sequences are described in U.S. Pat. Pub. No. 2011/0059865 A1. The standard P5 and P7 primer sequences for the paired-end sequencing comprise the following:
P5: paired end 5'→ 3'
   AATGATACGGCGACCACCGAGAUCTACAC (SEQ ID NO. 1)
P7: paired end 5'→ 3'
   CAAGCAGAAGACGGCATACGAG*AT (SEQ ID NO. 2)
where G* is 8-oxo-guanine.

Optionally, one or both of the P5 and P7 primers can include a poly T tail. The poly T tail is generally located at the 5' end of the above sequences, but in some cases can be located at the 3' end. The poly T sequence can include any number of T nucleotides, for example, from 2 to 20.

The standard P5 and P7 primer sequences used on a PAZAM coated flow cell with a poly-T spacer comprise the following:
P5 primer with poly-T spacer:
   5'-alkyne-TTTTTTTTTTAATGATACGGCGACCACCGAGAUCTACAC (SEQ ID NO. 3)
P7 primer with poly-T spacer:
   5'-alkyne-TTTTTTTTTTCAAGCAGAAGACGGCATACGAG*AT (SEQ ID NO: 4)
where G* is 8-oxo-guanine.

Additional primer sequences include a set of P5 and P7 primers for single read SBS:
P5: single read: 5'→ 3'
   AATGATACGGCGACCACCGA (SEQ ID NO. 5)
P7: single read 5'→ 3'
   CAAGCAGAAGACGGCATACGA (SEQ ID NO. 6)

As used herein, when the standard P5/P7 primers or oligos are modified to incorporate a first or second cleavage site that is capable of undergoing chemical cleavage, for example, by a Pd complex, the modification of the P5/P7 primers may refer to the replacement or substitution of an existing nucleotide (or nucleoside) in the P5/P7 sequence with a different chemical entity, for example, a modified nucleotide or nucleoside analogue with specific functionality to enable site-specific chemical cleavage. The modification may also refer to the insertion of a new chemical entity into the existing P5/P7 sequence, where the new chemical entity is capable of undergoing site specific chemical cleavage. In some embodiments, the modified P5/P7 primers are referred to as P15/P17 primers respectively, which are disclosed in U.S. Publication No. 2019/0352327. In particular, P15/P17 primers may comprise the following:
P15: 5'→ 3'
   AATGATACGGCGACCACCGAGA**T***CTACAC (SEQ. ID. NO. 7)
P17 primer 5'→ 3'
   **YYY**CAAGCAGAAGACGGCATACGAGAT (SEQ ID NO. 8)
P15 primer 5'→ 3'
   5'-alkyne-TTTTTTAATGATACGGCGACCACCGAGA**T***CTACAC (SEQ ID NO. 9)
P17 primer 5'→ 3'
   5'-Alkyne-TTTTTT**YYY**CAAGCAGAAGACGGCATACGAGAT (SEQ ID NO. 10)
where T* is a vinyl substituted T nucleoside; and Y is a diol linker subject to chemical cleavage, for example, by oxidation with a reagent such as periodate, as disclosed in U.S. Publication No. 2012/0109634.

In some embodiments, the diol linker comprises a Formula (I) or (Ia) as described herein. In some embodiments, the vinyl substituted T nucleoside comprises a Formula (II) as described herein.

As used herein, the terms "nucleic acid" and "nucleotide" are intended to be consistent with their use in the art and to include naturally occurring species or functional analogs thereof. Particularly useful functional analogs of nucleic acids are capable of hybridizing to a nucleic acid in a sequence specific fashion or capable of being used as a template for replication of a particular nucleotide sequence. Naturally occurring nucleic acids generally have a backbone containing phosphodiester bonds. An analog structure can have an alternate backbone linkage including any of a variety of those known in the art. Naturally occurring nucleic acids generally have a deoxyribose sugar (e.g., found in deoxyribonucleic acid (DNA)) or a ribose sugar (e.g., found in ribonucleic acid (RNA)). A nucleic acid can contain nucleotides having any of a variety of analogs of these sugar moieties that are known in the art. A nucleic acid can include native or non-native nucleotides. In this regard, a native deoxyribonucleic acid can have one or more bases selected from the group consisting of adenine, thymine, cytosine or guanine and a ribonucleic acid can have one or more bases selected from uracil, adenine, cytosine or guanine. Useful non-native bases that can be included in a nucleic acid or nucleotide are known in the art. The terms "probe" or "target," when used in reference to a nucleic acid, are intended as semantic identifiers for the nucleic acid in the context of a method or composition set forth herein and does not necessarily limit the structure or function of the nucleic acid beyond what is otherwise explicitly indicated. The terms "probe" and "target" can be similarly applied to other analytes such as proteins, small molecules, cells or the like.

As used herein, the term "polynucleotide" refers to nucleic acids in general, including DNA (e.g., genomic DNA cDNA), RNA (e.g., mRNA), synthetic oligonucleotides and synthetic nucleic acid analogs. Polynucleotides may include natural or non-natural bases, or combinations thereof and natural or non-natural backbone linkages, e.g., phosphorothioates, PNA or 2'-O-methyl-RNA, or combinations thereof. In some instances, the term "polynucleotide," "oligonucleotide," or "oligo" are used interchangeably.

The term "cleavage site" as used herein refers to a position on the polynucleotide sequence where a portion of the polynucleotide may be removed by a cleavage reaction. The position of the cleavage site is preferably pre-determined, meaning the location where the cleavage reaction happens is determined in advance, as opposed to cleavage at a random site where the location of which is not known in advance.

As used herein, the term "solid support" refers to a rigid substrate that is insoluble in aqueous liquid. The substrate can be non-porous or porous. The substrate can optionally be capable of taking up a liquid (e.g., due to porosity) but will typically be sufficiently rigid that the substrate does not swell substantially when taking up the liquid and does not contract substantially when the liquid is removed by drying. A nonporous solid support is generally impermeable to liquids or gases. Exemplary solid supports include, but are not limited to, glass and modified or functionalized glass, plastics (e.g., acrylics, polystyrene and copolymers of styrene and other materials, polypropylene, polyethylene, polybutylene, polyurethanes, Teflon^{™}, cyclic olefins, polyimides, etc.), nylon, ceramics, resins, Zeonor, silica or silica-based materials including silicon and modified silicon, carbon, metals, inorganic glasses, optical fiber bundles, and polymers. Particularly useful solid supports for some embodiments are components of a flow cell or located within a flow cell apparatus. The solid support may have a planar surface, for example, a flow cell, or a non-planar surface, for example, a bead.

Wherever a substituent is depicted as a di-radical (*i.e.,* has two points of attachment to the rest of the molecule), it is to be understood that the substituent can be attached in any directional configuration unless otherwise indicated. Thus, for example, a substituent depicted as -AE- or 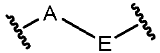 includes the substituent being oriented such that the A is attached at the leftmost attachment point of the molecule as well as the case in which A is attached at the rightmost attachment point of the molecule.

As used herein, a "nucleotide" includes a nitrogen containing heterocyclic base, a sugar, and one or more phosphate groups. They are monomeric units of a nucleic acid sequence. In RNA, the sugar is a ribose, and in DNA a deoxyribose, *i.e.* a sugar lacking a hydroxy group that is present in ribose. The nitrogen containing heterocyclic base can be purine or pyrimidine base. Purine bases include adenine (A) and guanine (G), and modified derivatives or analogs thereof, such as 7-deaza adenine or 7-deaza guanine. Pyrimidine bases include cytosine (C), thymine (T), and uracil (U), and modified derivatives or analogs thereof. The C-1 atom of deoxyribose is bonded to N-1 of a pyrimidine or N-9 of a purine.

As used herein, a "nucleoside" is structurally similar to a nucleotide, but is missing the phosphate moieties. An example of a nucleoside analogue would be one in which the label is linked to the base and there is no phosphate group attached to the sugar molecule. The term "nucleoside" is used herein in its ordinary sense as understood by those skilled in the art. Examples include, but are not limited to, a ribonucleoside comprising a ribose moiety and a deoxyribonucleoside comprising a deoxyribose moiety. A modified pentose moiety is a pentose moiety in which an oxygen atom has been replaced with a carbon and/or a carbon has been replaced with a sulfur or an oxygen atom. A "nucleoside" is a monomer that can have a substituted base and/or sugar moiety. Additionally, a nucleoside can be incorporated into larger DNA and/or RNA polymers and oligomers.

The term "purine base" is used herein in its ordinary sense as understood by those skilled in the art, and includes its tautomers. Similarly, the term "pyrimidine base" is used herein in its ordinary sense as understood by those skilled in the art, and includes its tautomers. A non-limiting list of optionally substituted purine-bases includes purine, adenine, guanine, deazapurine, 7-deaza adenine, 7-deaza guanine, hypoxanthine, xanthine, alloxanthine, 7-alkylguanine (e.g., 7-methylguanine), theobromine, caffeine, uric acid and isoguanine. Examples of pyrimidine bases include, but are not limited to, cytosine, thymine, uracil, 5,6-dihydrouracil and 5-alkylcytosine (e.g., 5-methylcytosine).

As used herein, "derivative" or "analog" means a synthetic nucleotide or nucleoside derivative having modified base moieties and/or modified sugar moieties. Such derivatives and analogs are discussed in, e.g., Scheit, Nucleotide Analogs (John Wiley & Son, 1980) and Uhlman et al., Chemical Reviews 90:543-584, 1990. Nucleotide analogs can also comprise modified phosphodiester linkages, including phosphorothioate, phosphorodithioate, alkyl-phosphonate, phosphoranilidate and phosphoramidate linkages. "Derivative", "analog" and "modified" as used herein, may be used interchangeably, and are encompassed by the terms "nucleotide" and "nucleoside" defined herein.

### Periodate Compositions for Diol Cleavage

Some embodiments of the present disclosure relate to an aqueous composition for chemical linearization of double-stranded polynucleotides, comprising: a periodate salt and an ionic liquid additive, wherein the periodate salt does not form a precipitate in the aqueous solution. In other words, the aqueous composition does not contain any precipitate of the periodate salt. As described herein, when a precipitate is not form, it means the precipitate cannot be observed in the aqueous solution. In further embodiments, the precipitate is not formed after the composition is subject to at least 2, 3, 4, 5, 6, 7, 8, 9, 10, 15 or 20 freeze/thaw cycles. In further embodiments, the precipitate may be less than 1%, 0.9%, 0.8%, 0.7%, 0.6%, 0.5%, 0.4%, 0.3%, 0.2%, 0.1%, 0.09%, 0.08%, 0.07%, 0.06%, 0.05%, 0.04%, 0.03%, 0.02%, 0.01%, 0.005%, or 0.001% by weight of the total amount of periodate salt in the composition.

In some embodiments of the composition described herein, the concentration of the periodate salt in the composition is from about 0.1 mM to about 300 mM, from about 0.5 mM to about 200 mM, from about 1 mM to about 150 mM, from about 2 mM to about 100 mM, or from about 5 mM to about 50 mM. In further embodiments, the concentration of the periodate salt in the composition is about 1 mM, 2 mM, 3 mM, 4 mM, 5 mM, 6 mM, 7 mM, 8 mM, 9 mM, 10 mM, 12 mM, 14 mM, 16 mM, 18 mM, 20 mM or 25 mM, or a range defined by any two of the preceding values. In one embodiment, the concentration of the periodate salt in the composition is about 10 mM. In another embodiment, the concentration of the periodate salt in the composition is about 25 mM.

In some embodiments of the composition described herein, the ionic liquid additive comprises or is a crown ether or a substituted imidazolium salt, or a combination thereof. In some such embodiments, the crown ether comprises or is 15-crown-5, having the structure 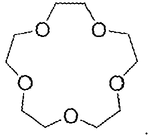 In some embodiments, the molar ratio of the crown ether (e.g. 15-crown-5) to the periodate salt in the composition is from about 1:1 to about 50:1 or from about 5:1 to about 25:1. In further embodiments, the molar ratio of the crown ether (e.g. 15-crown-5) to the periodate salt in the composition is about 2:1, 3:1, 4:1, 5:1, 6:1, 7:1, 8:1, 9:1, 10:1, 12:1, 14:1, 16:1, 18:1, 20:1, 25:1 or 30:1. In one embodiment, the molar ratio of the crown ether (e.g. 15-crown-5) to the periodate salt is about 10:1. In other embodiments, the substituted imidazolium salt comprises or is a substituted imidazolium halide salt, e.g., 1-benzyl-3-methylimidazolium chloride ([Bzmim]Cl, having the structure 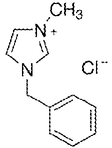 ). In some embodiments, the molar ratio of the substituted imidazolium salt (e.g., [Bzmim]Cl) to the periodate salt in the composition is from about 1:1 to about 100:1, from about 5:1 to about 75:1, or from about 10:1 to about 50:1. In further embodiments, the molar ratio of the substituted imidazolium salt (e.g., [Bzmim]Cl) to the periodate salt is about 5:1, 10:1, 15:1, 20:1, 25:1, 30:1, 35:1, 40:1, 45:1 or 50:1, or a range defined by any two of the preceding values. In one embodiment, the molar ratio of the substituted imidazolium salt (e.g., [Bzmim]Cl) to the periodate salt is about 30:1.

In some embodiments of the composition described herein, the composition further comprises one or more inorganic salts. The presence of these inorganic salts may increase the ionic strength of the composition, and results in an increased linearization rate. For example, the inorganic salt may comprise one or more sodium salts, or one or more magnesium salts, or a combination thereof. In further embodiments, the inorganic salt may comprise sodium citrate, sodium acetate, magnesium sulfate, or combinations thereof.

In some embodiments of the composition described herein, the pH of the composition is from about 4 to about 8, about 5 to about 7.5, or about 6. In one embodiment, the pH of the composition is about 5.2. In another embodiment, the pH of the composition is about 6.0.

In any embodiments of the composition described herein, the periodate salt comprises or is sodium periodate.

The periodate composition described herein may also improve the chemical linearization rate of the double-stranded polynucleotides as compared to the chemical linearization rate using the same periodate composition without the ionic liquid additive. For example, the chemical linearization rate may be increased by at least about 20%, 50%, 100%, 150%, 200%, 250%, or 300%, compared to the same method using the same periodate salt composition without the ionic liquid additive (e.g., [Bzmim]Cl). A particular embodiment of the periodate composition described herein comprise sodium periodate and [Bzmim]Cl, wherein the concentration of sodium periodate in the composition is from about 5mM to about 20 mM, or about 10 mM. In further embodiments, the concentration of [Bzmim]Cl in the concentration is from about 150 mM to 600 mM, or about 300 mM. The pH of the composition is from about 4 to about 8, or about 5.2, or about 6.

### Methods of Chemical Linearization with A Periodate Salt

Another aspect of the present disclosure relates to a method of linearizing a plurality of immobilized double-stranded polynucleotides, comprising:
contacting a composition containing a periodate salt as described herein with a solid support comprising the plurality of immobilized double-stranded polynucleotides, each double-stranded polynucleotide comprises a first strand and a second strand, wherein the first strand and the second strand are immobilized to the solid support at their 5' ends, wherein each second strand comprises a cleavage site; and
chemically cleaving one or more second strands at the cleavage site, and generating one or more cleaved second nucleic acids and cleaved immobilized second strands.

In some embodiments of the periodate linearization method described herein, the method further comprises removing the cleaved second nucleic acids from the solid support.

In some embodiments of the periodate linearization method described herein, each second strand is extended from a second extension primer immobilized to the solid support, and each second extension primer comprises the cleavage site.

In some embodiments of the periodate linearization method described herein, the cleavage site of each second strand comprises one or more diol linkers. In some embodiments, the diol linker comprises a structure of Formula (I): 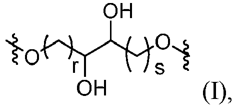 wherein r is 2, 3, 4, 5, or 6; and s is 2, 3, 4, 5, or 6. In further embodiments, the diol linker comprises or has the structure: 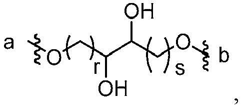 where the "a" oxygen is the 3' hydroxyl oxygen of a first nucleotide; and the "b" oxygen is the 5' hydroxyl oxygen of a second nucleotide.

In one embodiment, the diol linker comprises a structure of Formula (Ia): 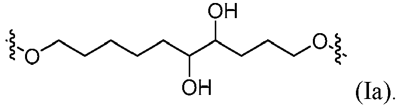 In a further embodiment, the diol linker comprises or has a structure of 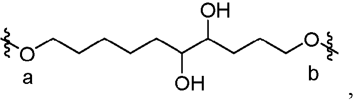 where the "a" oxygen is the 3' hydroxyl oxygen of a first nucleotide; and the "b" oxygen is the 5' hydroxyl oxygen of a second nucleotide. In a further embodiment, the second extension primer comprises a P17 primer (SEQ ID NO. 8 or 10). In one embodiment, the second extension primer is a P17 primer.

In some embodiments of the periodate linearization method described herein, the periodate composition described herein may also improve the chemical linearization rate of the double-stranded polynucleotides as compared to the chemical linearization rate using the same periodate composition without the ionic liquid additive. For example, the chemical linearization rate may be increased by at least about 20%, 50%, 100%, 150%, 200%, 250%, or 300%, compared to the same method using the same periodate salt composition without the ionic liquid additive (e.g., [Bzmim]Cl). In some embodiments, the molar ratio of the substituted imidazolium salt (e.g., [Bzmim]Cl) to the periodate salt is about 5:1, 10:1, 15:1, 20:1, 25:1, 30:1, 35:1, 40:1, 45:1 or 50:1, or a range defined by any two of the preceding values. In one embodiment, the molar ratio of the substituted imidazolium salt (e.g., [Bzmim]Cl) to the periodate salt is about 30:1.

### Methods of Transition Metal Catalyzed Chemical Linearization

Some embodiments of the present disclosure relate to a method of linearizing a plurality of immobilized double-stranded polynucleotides, comprising: providing a solid support comprising double-stranded polynucleotides, wherein each double-stranded polynucleotide comprises a first strand and a second strand, wherein the first strand and the second strand are each immobilized to the solid support at their 5' ends, and wherein each first strand comprises a first cleavage site capable of undergoing chemical cleavage in the presence of a cleavage reagent (e.g., a transition metal catalyst); contacting the double-stranded polynucleotides with the cleavage reagent, thereby cleaving one or more first strands at the first cleavage site, and generating one or more cleaved first nucleic acids and cleaved immobilized first strands. In some embodiments, the method further and removing the cleaved first nucleic acids from the solid support. In some aspect, the cleavage site is capable of undergoing chemical cleavage in the presence of a Pd complex (e.g., a Pd(0) complex). In some aspect, the cleavage reagent is an aqueous solution of the Pd complex. In some aspect, the cleavage reagent (e.g., a Pd(0) complex) is prepared *in situ.*

In some embodiments of the Pd catalyzed linearization method described herein, each first strand is extended from a first extension primer immobilized to the solid support.

In some embodiments of the Pd linearization methods described herein, the first extension primer comprises the first cleavage site. In some further embodiments, the first cleavage site comprises a modified nucleoside/nucleotide that is capable of undergoing chemical cleavage, for example by the palladium complex. In some embodiments, the first cleavage site incorporating the modified nucleoside/nucleotide moiety comprises the structure of Formula (II), where the 3' oxygen of the vinyl substituted nucleoside or nucleotide is covalently attached to the 5' end of another nucleotide (structure not shown): 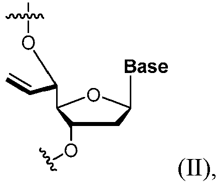 wherein Base is adenine, 7-deazaademine, guanine, 7-deazaguanine, cytosine, thymine, or uracil, or an analog or derivative thereof. In some embodiments, the modified nucleotide or nucleoside is a thymine (T) nucleoside or nucleotide analogue. In some embodiments, the cleavage site is located near the 3' end of the first extension primer, for example, within 1, 2, 3, 4, 5, 6, 7, 8, 9, or 10 nucleotide distance from the 3' end of the first extension primer. In some other embodiments, the cleavage site is located near the 5' end of the first extension primer, for example, within 1, 2, 3, 4, 5, 6, 7, 8, 9, or 10 nucleotide distance from the 5' end of the first extension primer. In some cases, to ensure efficient DNA resynthesis, the cleavage site is preferably located towards the 3' end of the first primer, for example, within 2 to 8, or 3 to 7, or 4 to 6 nucleotide distance. In one embodiment, the first extension primer is a P5 primer, and the first cleavage site is located in the P5 primer sequence (e.g., the modified nucleotide is incorporated into the P5 primer sequence, by adding to or replacing one nucleotide). Therefore, the P5 sequence disclosed herein (SEQ ID NO. 1 or SEQ ID NO. 3) is modified to include the first cleavage site that is capable of undergoing chemical cleavage by the Pd/Ni complex, thus forming a modified P5 primer. In one embodiment, the modified P5 primer comprises or is a P15 primer disclosed herein (SEQ ID NO. 7 or 9).

### Palladium Reagents

In some embodiments of the Pd linearization methods described herein, the Pd complex used in the chemical linearization method is water soluble. In some such embodiments, the Pd complex is a Pd(0) complex. In some instances, the Pd(0) complex may be generated *in situ* from reduction of a Pd(II) complex by reagents such as alkenes, alcohols, amines, phosphines, or metal hydrides. Suitable palladium sources include Na₂PdCl₄, (PdCl(C₃H₅))₂, [Pd(C₃H₅)(THP)]Cl, [Pd(C₃H₅)(THP)₂]Cl, Pd(OAc)₂, Pd(Ph₃)₄, Pd(dba)₂, and Pd(TFA)₂. In one such embodiment, the Pd(0) complex is generated *in situ* from Na₂PdCl₄. In another embodiment, the palladium source is allyl palladium(II) chloride dimer [(PdCl(C₃H₅))₂]. In some embodiments, the Pd(0) complex is generated in an aqueous solution by mixing a Pd(II) complex with a phosphine. Suitable phosphines include water soluble phosphines, such as tris(hydroxypropyl)phosphine (THP), tris(hydroxymethyl)phosphine (THM), 1,3,5-triaza-7-phosphaadamantane (PTA), bis(p-sulfonatophenyl)phenylphosphine dihydrate potassium salt, tris(carboxyethyl)phosphine (TCEP), and triphenylphosphine-3,3',3''-trisulfonic acid trisodium salt.

In some embodiments, the Pd complex is a Pd(II) complex (e.g., Pd(OAc)₂, [(Allyl)PdCl]₂ or Na₂PdCl₄), which generates Pd(0) *in situ* in the presence of the phosphine (e.g., THP). For example, the Pd(0) catalyst may be prepared by mixing a Pd(II) complex [(PdCl(C₃H₅))₂] with THP *in situ.* The molar ratio of the Pd(II) complex and the THP may be about 1:2, 1:3, 1:4, 1:5, 1:6, 1:7, 1:8, 1:9, or 1:10. In some further embodiments, one or more reducing agents may be added, such as ascorbic acid or a salt thereof (e.g., sodium ascorbate). In some other embodiments, the Pd(0) is prepared by mixing a Pd(II) pre-catalyst such as [Pd(C₃H₅)(THP)]Cl, [Pd(C₃H₅)(THP)₂]Cl with additional THP. [Pd(C₃H₅)(THP)]Cl and [Pd(C₃H₅)(THP)₂]Cl may be prepared by reacting (PdCl(C₃H₅))₂ with 1 to 5 equivalents of THP and they may be isolated prior to use in the chemical linearization reaction.

### Denaturation

In any embodiments of method used for cleavage, the product of the cleavage reaction may be subjected to denaturing conditions in order to remove the portion(s) of the cleaved strand(s) that are not attached to the solid support. Suitable denaturing conditions will be apparent to the skilled reader with reference to standard molecular biology protocols (Sambrook et al., 2001, Molecular Cloning, A Laboratory Manual, 3rd Ed, Cold Spring Harbor Laboratory Press, Cold Spring Harbor Laboratory Press, NY; Current Protocols, eds., Ausubel et al.). Denaturation (and subsequent re-annealing of the cleaved strands) results in the production of a sequencing template which is partially or substantially single-stranded. A sequencing reaction may then be initiated by hybridization of a sequencing primer to the single-stranded portion of the template.

In other embodiments, sequencing can be initiated directly after the cleavage step with no need for denaturation to remove a portion of the cleaved strand(s). If the cleavage step generates a free 3' hydroxyl group on one cleaved strand still hybridized to a complementary strand, then sequencing can proceed from this point using a strand-displacement polymerase enzyme without the need for an initial denaturation step. In particular, strand displacement sequencing may be used in conjunction with template generation by cleavage with nicking endonucleases, or by hydrolysis of an abasic site with endonuclease, heat or alkali treatment.

### Methods of Sequencing

Another aspect of the present disclosure relates to a method of sequencing polynucleotides, comprising:
contacting a first linearization reagent with a solid support comprising a plurality of immobilized double-stranded polynucleotides, each double-stranded polynucleotide comprises a first strand and a second strand, wherein the first strand and the second strand are immobilized to the solid support at their 5' ends, wherein each first strand comprises a first cleavage site, and wherein each second strand comprises a second cleavage site comprising one or more diol linkers as described herein;
cleaving one or more first strands at the first cleavage site with the first linearization reagent, and generating one or more cleaved first nucleic acids and cleaved immobilized first strands;
sequencing the immobilized second strands;
resynthesizing derivative first strands that are complementary to the second strands;
contacting a composition containing a periodate salt as described herein with one or more second strands to cleave the second strands at the second cleavage site, and generating one or more cleaved second nucleic acids and cleaved immobilized second strands; and
sequencing the immobilized derivative first strands.

In some embodiments of the sequencing method described herein, the method further comprises protecting any free 3' hydroxy group of the cleaved immobilized first strands with a 3' hydroxy blocking group prior to sequencing the immobilized second strands. In some embodiments, the method further comprises removing the one or more cleaved first nucleic acids from the solid support before sequencing the immobilized second strands. In some embodiments, the method further comprises removing the one or more cleaved second nucleic acids from the solid support before sequencing the immobilized derivative first strands.

In some embodiments of the sequencing method described herein, the sequencing of the immobilized second strands is done through sequencing-by-synthesis (SBS), which is described in detail below. In some further embodiments, the method further comprises a denature step to remove the complementary strands formed by the SBS of the immobilized second strands, before resyntheses of the immobilized derivative first strands start. In further embodiments, the method further comprises deprotecting the 3' hydroxy blocking group of the cleaved immobilized first strands before the resynthesis step. In further embodiments, the sequencing of the immobilized derivative first strands is also done through SBS.

In some embodiments of the sequencing method described herein, the first linearization reagent comprises or is a chemical linearization reagent, such as a palladium catalyst (e.g., a Pd(0) complex described herein). This step of linearization is also called the first chemical cleavage linearization. The step of the periodate salt cleavage of the diol linker at the second cleavage site is also called the second chemical cleavage linearization.

In some embodiments of the sequencing method described herein, the first cleavage site comprises a modified nucleotide comprising a structure of Formula (II): 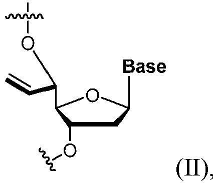 wherein Base is adenine, 7-deazaademine, guanine, 7-deazaguanine, cytosine, thymine, or uracil, or a derivative thereof. In further embodiments, each first strand is extended from a first extension primer immobilized to the solid support, and wherein the first extension primer comprises a P15 sequence. In some embodiments, the diol linker comprises a structure of Formula (I): 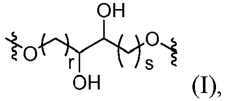 wherein r is 2, 3, 4, 5, or 6; and s is 2, 3, 4, 5, or 6. In one embodiment, the diol linker comprises a structure of Formula (Ia): 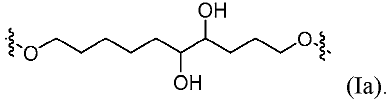 In further embodiments, each second strand is extended from a second extension primer immobilized to the solid support, and wherein the second extension primer comprises a P17 sequence.

In other embodiments, the first cleavage site may be cleaved by a method selected from the group consisting of photo cleavage, enzymatic cleavage, or a combination thereof. In one embodiment, the first cleavage site may be cleaved by an enzymatic cleavage reaction. In one embodiment, the first extension primer is a P5 primer disclosed herein (SEQ ID NO. 1 or 3), containing a deoxyuridine (U) that can be enzymatically cleaved by enzyme USER.

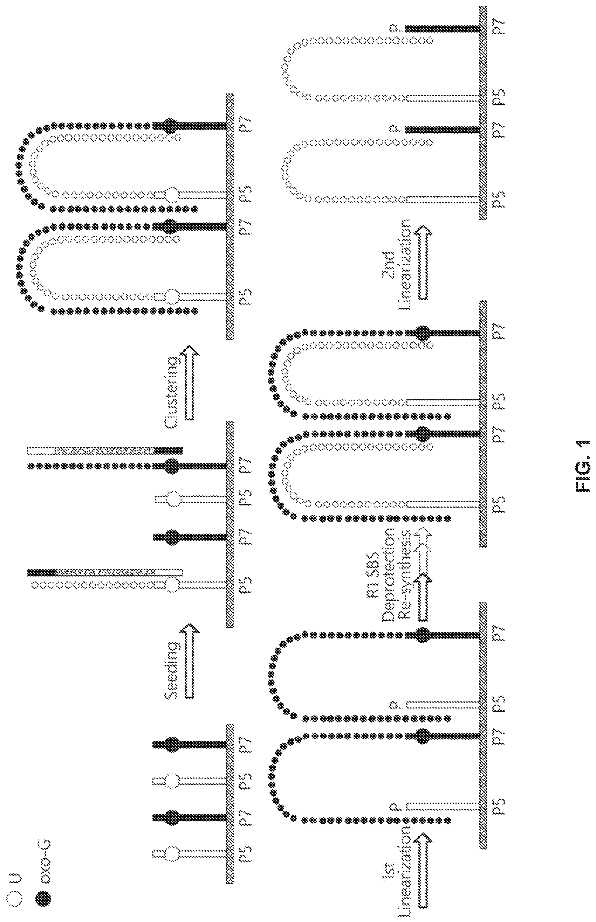

**FIG. 1** describes an embodiment of a standard workflow of the Illumina SBS chemistry. First, a solid support comprising a plurality of P5/P7 primers immobilized on the surface of the solid support is provided. Each of the P5 and the P7 primers has a cleavage site within the sequence. In one embodiment, the cleavage site on the P5 primer is a deoxyuridine (U). In one embodiment, the cleavage site on the P7 primer is an 8-oxo-guanine nucleotide (oxo-G). A set of target DNA molecules to be sequenced is hybridized to the immobilized P5/P7 primers. After hybridization, the original target DNA molecules are removed, leaving only the complementary copies of the extended polynucleotides containing the P5/P7 primers. This step is also known as a "seeding" step. Then, the extended P5/P7 polynucleotides are amplified through a process called the "bridge amplification," forming double-stranded clusters with both strands being attached to the solid support at the 5' end. After the "clustering" step, the first linearization is performed to remove a portion of the extended polynucleotides containing the P5 primer. In one embodiment, such removal is facilitated by an enzymatic cleavage reaction using an enzyme USER to cleave the U position on the P5 primer (first linearization step). After a first round of SBS (Read 1), a resynthesis is carried out to form the double-stranded polynucleotides again. Then, a second linearization is performed to remove a portion of the extended polynucleotides containing the P7 primer. In one embodiment, such removal is facilitated by an enzymatic cleavage reaction using enzyme FPG to cleave the oxo-G position of the P7 primer. Then a second round of SBS is carried out (Read 2) to sequence the target DNA.

Alternatively, the P5 primer may be replaced by P15 primer and the P7 primer may be replaced by P17 primer. In this case, the first linearization step may be achieved by a chemical cleavage linearization using a Pd catalyst described herein. The second linearization step may also be achieved by a chemical cleavage linearization using the periodate composition (e.g., sodium periodate) described herein.

Alternatively, the solid support may comprise P5/P17 primers. In this case, the first linearization step may be achieved by an enzymatic linearization using USER. The second linearization step may be achieved by a chemical cleavage linearization using the periodate composition (e.g., sodium periodate) described herein.

In some embodiments, the methods described herein can be used for determining a nucleotide sequence of a polynucleotide. In such embodiments, the method can comprise the steps of (a) contacting a polynucleotide polymerase with delinearized polynucleotide (also described below as target polynucleotide) clusters attached to a surface of a substrate (e.g., via any one of the polymer or gel coatings described herein); (b) providing nucleotides to the surface of the substrate such that a detectable signal is generated when one or more nucleotides are utilized by the polynucleotide polymerase; (c) detecting signals at one or more attached polynucleotide (or one or more clusters produced from the attached polynucleotides); and (d) repeating steps (b) and (c), thereby determining a nucleotide sequence of a substrate-attached polynucleotide.

Labeled nucleotides may be used in any method of analysis such as method that include detection of a fluorescent label attached to such nucleotide, whether on its own or incorporated into or associated with a larger molecular structure or conjugate. In this context the term "incorporated into a polynucleotide" can mean that the 5' phosphate is joined in phosphodiester linkage to the 3' hydroxy group of a second nucleotide, which may itself form part of a longer polynucleotide chain. The 3' end of a nucleotide set forth herein may or may not be joined in phosphodiester linkage to the 5' phosphate of a further nucleotide. Thus, in one non-limiting embodiment, the disclosure provides a method of detecting a labeled nucleotide incorporated into a polynucleotide which comprises: (a) incorporating at least one labeled nucleotide of the disclosure into a polynucleotide and (b) determining the identity of the nucleotide(s) incorporated into the polynucleotide by detecting the fluorescent signal from the dye compound attached to said nucleotide(s).

This method can include: a synthetic step (a) in which one or more labeled nucleotides according to the disclosure are incorporated into a single stranded polynucleotide and a detection step (b) in which one or more labeled nucleotide(s) incorporated into the polynucleotide are detected by detecting or quantitatively measuring their fluorescence.

Some embodiments of the present application are directed to a method of determining the sequence of a target polynucleotide (e.g., a single-stranded target polynucleotide), comprising: (a) contacting a primer polynucleotide with one or more labeled nucleotides (such as nucleoside triphosphates A, G, C and T), and wherein the primer polynucleotide is complementary to at least a portion of the target polynucleotide; (b) incorporating a labeled nucleotide into the primer polynucleotide; and (c) performing one or more fluorescent measurements to determine the identify of the incorporated nucleotide. In some such embodiments, the primer polynucleotide/target polynucleotide complex is formed by contacting the target polynucleotide with a primer polynucleotide complementary to at least a portion of the target polynucleotide. In some embodiments, the method further comprises (d) removing the label moiety and the 3' hydroxy blocking group from the nucleotide incorporated into the primer polynucleotide. In some further embodiments, the method may also comprises (e) washing the removed label moiety and the 3' blocking group away from the primer polynucleotide strand. In some embodiments, steps (a) through (d) or steps (a) through (e) are repeated until a sequence of at least a portion of the target polynucleotide strand is determined. In some instances, steps (a) through (d) or steps (a) through (e) are repeated at least at least 30, 40, 50, 60, 70, 80, 90, 100, 110, 120, 130, 140, 150, 160, 170, 180, 190, 200, 250, or 300 cycles. In some embodiments, the label moiety and the 3' blocking group from the nucleotide incorporated into the primer polynucleotide strand are removed in a single chemical reaction. In some further embodiments, the method is performed on an automated sequencing instrument, and wherein the automated sequencing instrument comprises two light sources operating at different wavelengths. In some embodiments, the sequence determination is conducted after the completion of repeated cycles of the sequencing steps described herein.

In some embodiments, at least one nucleotide is incorporated into a polynucleotide (such as a single stranded primer polynucleotide described herein) in the synthetic step by the action of a polymerase enzyme. However, other methods of joining nucleotides to polynucleotides, such as, for example, chemical oligonucleotide synthesis or ligation of labeled oligonucleotides to unlabeled oligonucleotides, can be used. Therefore, the term "incorporating," when used in reference to a nucleotide and polynucleotide, can encompass polynucleotide synthesis by chemical methods as well as enzymatic methods.

In a specific embodiment, a synthetic step is carried out and may optionally comprise incubating a template or target polynucleotide strand with a reaction mixture comprising fluorescently labeled nucleotides of the disclosure. A polymerase can also be provided under conditions which permit formation of a phosphodiester linkage between a free 3' hydroxy group on a polynucleotide strand annealed to the template or target polynucleotide strand and a 5' phosphate group on the labeled nucleotide. Thus, a synthetic step can include formation of a polynucleotide strand as directed by complementary base pairing of nucleotides to a template/target strand.

In all embodiments of the methods, the detection step may be carried out while the polynucleotide strand into which the labeled nucleotides are incorporated is annealed to a template/target strand, or after a denaturation step in which the two strands are separated. Further steps, for example chemical or enzymatic reaction steps or purification steps, may be included between the synthetic step and the detection step. In particular, the polynucleotide strand incorporating the labeled nucleotide(s) may be isolated or purified and then processed further or used in a subsequent analysis. By way of example, polynucleotide strand incorporating the labeled nucleotide(s) as described herein in a synthetic step may be subsequently used as labeled probes or primers. In other embodiments, the product of the synthetic step set forth herein may be subject to further reaction steps and, if desired, the product of these subsequent steps purified or isolated.

Suitable conditions for the synthetic step will be well known to those familiar with standard molecular biology techniques. In one embodiment, a synthetic step may be analogous to a standard primer extension reaction using nucleotide precursors, including the labeled nucleotides as described herein, to form an extended polynucleotide strand (primer polynucleotide strand) complementary to the template/target strand in the presence of a suitable polymerase enzyme. In other embodiments, the synthetic step may itself form part of an amplification reaction producing a labeled double stranded amplification product comprised of annealed complementary strands derived from copying of the primer and template polynucleotide strands. Other exemplary synthetic steps include nick translation, strand displacement polymerization, random primed DNA labeling, etc. A particularly useful polymerase enzyme for a synthetic step is one that is capable of catalyzing the incorporation of the labeled nucleotides as set forth herein. A variety of naturally occurring or mutant/modified polymerases can be used. By way of example, a thermostable polymerase can be used for a synthetic reaction that is carried out using thermocycling conditions, whereas a thermostable polymerase may not be desired for isothermal primer extension reactions. Suitable thermostable polymerases which are capable of incorporating the labeled nucleotides according to the disclosure include those described in WO 2005/024010 or WO06120433. In synthetic reactions which are carried out at lower temperatures such as 37 °C, polymerase enzymes need not necessarily be thermostable polymerases, therefore the choice of polymerase will depend on a number of factors such as reaction temperature, pH, strand-displacing activity and the like.

In specific non-limiting embodiments, the disclosure encompasses methods of nucleic acid sequencing, re-sequencing, whole genome sequencing, single nucleotide polymorphism scoring, any other application involving the detection of the modified nucleotide or nucleoside labeled with dyes set forth herein when incorporated into a polynucleotide.

A particular embodiment of the disclosure provides use of labeled nucleotides comprising dye moiety according to the disclosure in a polynucleotide sequencing-by-synthesis reaction. Sequencing-by-synthesis generally involves sequential addition of one or more nucleotides or oligonucleotides to a growing polynucleotide chain in the 5' to 3' direction using a polymerase or ligase in order to form an extended polynucleotide chain complementary to the template/target nucleic acid to be sequenced. The identity of the base present in one or more of the added nucleotide(s) can be determined in a detection or "imaging" step. The identity of the added base may be determined after each nucleotide incorporation step. The sequence of the template may then be inferred using conventional Watson-Crick base-pairing rules. The use of the nucleotides labeled with dyes set forth herein for determination of the identity of a single base may be useful, for example, in the scoring of single nucleotide polymorphisms, and such single base extension reactions are within the scope of this disclosure.

In an embodiment of the present disclosure, the sequence of a template/target polynucleotide is determined by detecting the incorporation of one or more nucleotides into a nascent strand complementary to the template polynucleotide to be sequenced through the detection of fluorescent label(s) attached to the incorporated nucleotide(s). Sequencing of the template polynucleotide can be primed with a suitable primer (or prepared as a hairpin construct which will contain the primer as part of the hairpin), and the nascent chain is extended in a stepwise manner by addition of nucleotides to the 3' end of the primer in a polymerase-catalyzed reaction.

In particular embodiments, each of the different nucleotide triphosphates (A, T, G and C) may be labeled with a unique fluorophore and also comprises a blocking group at the 3' position to prevent uncontrolled polymerization. Alternatively, one of the four nucleotides may be unlabeled (dark). The polymerase enzyme incorporates a nucleotide into the nascent chain complementary to the template/target polynucleotide, and the blocking group prevents further incorporation of nucleotides. Any unincorporated nucleotides can be washed away and the fluorescent signal from each incorporated nucleotide can be "read" optically by suitable means, such as a charge-coupled device using light source excitation and suitable emission filters. The 3' blocking group and fluorescent dye compounds can then be removed (deprotected) (simultaneously or sequentially) to expose the nascent chain for further nucleotide incorporation. Typically, the identity of the incorporated nucleotide will be determined after each incorporation step, but this is not strictly essential. Similarly, U.S. Pat. No. 5,302,509 discloses a method to sequence polynucleotides immobilized on a solid support.

The method, as exemplified above, utilizes the incorporation of fluorescently labeled, 3'-blocked nucleotides A, G, C, and T into a growing strand complementary to the immobilized polynucleotide, in the presence of DNA polymerase. The polymerase incorporates a base complementary to the target polynucleotide but is prevented from further addition by the 3'-blocking group. The label of the incorporated nucleotide can then be determined, and the blocking group removed by chemical cleavage to allow further polymerization to occur. The nucleic acid template to be sequenced in a sequencing-by-synthesis reaction may be any polynucleotide that it is desired to sequence. The nucleic acid template for a sequencing reaction will typically comprise a double stranded region having a free 3' hydroxy group that serves as a primer or initiation point for the addition of further nucleotides in the sequencing reaction. The region of the template to be sequenced will overhang this free 3' hydroxy group on the complementary strand. The overhanging region of the template to be sequenced may be single stranded but can be double-stranded, provided that a "nick is present" on the strand complementary to the template strand to be sequenced to provide a free 3' OH group for initiation of the sequencing reaction. In such embodiments, sequencing may proceed by strand displacement. In certain embodiments, a primer bearing the free 3' hydroxy group may be added as a separate component (e.g., a short oligonucleotide) that hybridizes to a single-stranded region of the template to be sequenced. Alternatively, the primer and the template strand to be sequenced may each form part of a partially self-complementary nucleic acid strand capable of forming an intra-molecular duplex, such as for example a hairpin loop structure. Hairpin polynucleotides and methods by which they may be attached to solid supports are disclosed in PCT Publication Nos. WO0157248 and WO2005/047301, each of which is incorporated herein by reference. Nucleotides can be added successively to a growing primer, resulting in synthesis of a polynucleotide chain in the 5' to 3' direction. The nature of the base which has been added may be determined, particularly but not necessarily after each nucleotide addition, thus providing sequence information for the nucleic acid template. Thus, a nucleotide is incorporated into a nucleic acid strand (or polynucleotide) by joining of the nucleotide to the free 3' hydroxy group of the nucleic acid strand via formation of a phosphodiester linkage with the 5' phosphate group of the nucleotide.

The nucleic acid template to be sequenced may be DNA or RNA, or even a hybrid molecule comprised of deoxynucleotides and ribonucleotides. The nucleic acid template may comprise naturally occurring and/or non-naturally occurring nucleotides and natural or non-natural backbone linkages, provided that these do not prevent copying of the template in the sequencing reaction.

In certain embodiments, the nucleic acid template to be sequenced may be attached to a solid support via any suitable linkage method known in the art, for example via covalent attachment. In certain embodiments template polynucleotides may be attached directly to a solid support (e.g., a silica-based support). However, in other embodiments of the disclosure the surface of the solid support may be modified in some way so as to allow either direct covalent attachment of template polynucleotides, or to immobilize the template polynucleotides through a hydrogel or polyelectrolyte multilayer, which may itself be non-covalently attached to the solid support.

Arrays in which polynucleotides have been directly attached to a support (for example, silica-based supports such as those disclosed in WO00/06770, wherein polynucleotides are immobilized on a glass support by reaction between a pendant epoxide group on the glass with an internal amino group on the polynucleotide. In addition, polynucleotides can be attached to a solid support by reaction of a sulfur-based nucleophile with the solid support, for example, as described in WO2005/047301. A still further example of solid-supported template polynucleotides is where the template polynucleotides are attached to hydrogel supported upon silica-based or other solid supports, for example, as described in WO00/31148, WO01/01143, WO02/12566, WO03/014392, U.S. Pat. No. 6,465,178 and WO00/53812.

A particular surface to which template polynucleotides may be immobilized is a polyacrylamide hydrogel. Polyacrylamide hydrogels are described in the references cited above and in WO2005/065814. Specific hydrogels that may be used include those described in WO2005/065814 and U.S. Pub. No. 2014/0079923. In one embodiment, the hydrogel is PAZAM (poly(N-(5-azidoacetamidylpentyl) acrylamide-co-acrylamide)).

DNA template molecules can be attached to beads or microparticles, for example, as described in U.S. Pat. No. 6,172,218 Attachment to beads or microparticles can be useful for sequencing applications. Bead libraries can be prepared where each bead contains different DNA sequences. Exemplary libraries and methods for their creation are described in Nature, 437, 376-380 (2005); Science, 309, 5741, 1728-1732 (2005). Sequencing of arrays of such beads using nucleotides set forth herein is within the scope of the disclosure.

Template(s) that are to be sequenced may form part of an "array" on a solid support, in which case the array may take any convenient form. Thus, the method of the disclosure is applicable to all types of high-density arrays, including single-molecule arrays, clustered arrays, and bead arrays. Nucleotides labeled with dye compounds of the present disclosure may be used for sequencing templates on essentially any type of array, including but not limited to those formed by immobilization of nucleic acid molecules on a solid support.

However, nucleotides labeled with dye compounds of the disclosure are particularly advantageous in the context of sequencing of clustered arrays. In clustered arrays, distinct regions on the array (often referred to as sites, or features) comprise multiple polynucleotide template molecules. Generally, the multiple polynucleotide molecules are not individually resolvable by optical means and are instead detected as an ensemble. Depending on how the array is formed, each site on the array may comprise multiple copies of one individual polynucleotide molecule (e.g., the site is homogenous for a particular single- or double-stranded nucleic acid species) or even multiple copies of a small number of different polynucleotide molecules (e.g., multiple copies of two different nucleic acid species). Clustered arrays of nucleic acid molecules may be produced using techniques generally known in the art. By way of example, WO 98/44151 and WO00/18957, describe methods of amplification of nucleic acids wherein both the template and amplification products remain immobilized on a solid support in order to form arrays comprised of clusters or "colonies" of immobilized nucleic acid molecules. The nucleic acid molecules present on the clustered arrays prepared according to these methods are suitable templates for sequencing using nucleotides labeled with dye compounds of the disclosure.

Nucleotides labeled with dye compounds of the present disclosure are also useful in sequencing of templates on single molecule arrays. The term "single molecule array" or "SMA" as used herein refers to a population of polynucleotide molecules, distributed (or arrayed) over a solid support, wherein the spacing of any individual polynucleotide from all others of the population is such that it is possible to individually resolve the individual polynucleotide molecules. The target nucleic acid molecules immobilized onto the surface of the solid support can thus be capable of being resolved by optical means in some embodiments. This means that one or more distinct signals, each representing one polynucleotide, will occur within the resolvable area of the particular imaging device used.

Single molecule detection may be achieved wherein the spacing between adjacent polynucleotide molecules on an array is at least 100 nm, more particularly at least 250 nm, still more particularly at least 300 nm, even more particularly at least 350 nm. Thus, each molecule is individually resolvable and detectable as a single molecule fluorescent point, and fluorescence from said single molecule fluorescent point also exhibits single step photobleaching.

The terms "individually resolved" and "individual resolution" are used herein to specify that, when visualized, it is possible to distinguish one molecule on the array from its neighboring molecules. Separation between individual molecules on the array will be determined, in part, by the particular technique used to resolve the individual molecules. The general features of single molecule arrays will be understood by reference to published applications WO00/06770 and WO 01/57248. Although one use of the labeled nucleotides of the disclosure is in sequencing-by-synthesis reactions, the utility of the such nucleotides is not limited to such methods. In fact, the labeled nucleotides described herein may be used advantageously in any sequencing methodology which requires detection of fluorescent labels attached to nucleotides incorporated into a polynucleotide.

In particular, nucleotides labeled with dye compounds of the disclosure may be used in automated fluorescent sequencing protocols, particularly fluorescent dye-terminator cycle sequencing based on the chain termination sequencing method of Sanger and co-workers. Such methods generally use enzymes and cycle sequencing to incorporate fluorescently labeled dideoxynucleotides in a primer extension sequencing reaction. So-called Sanger sequencing methods, and related protocols (Sanger-type), utilize randomized chain termination with labeled dideoxynucleotides.

Thus, the present disclosure also encompasses nucleotides labeled with dye compounds which are dideoxynucleotides lacking hydroxy groups at both of the 3' and 2' positions, such modified dideoxynucleotides being suitable for use in Sanger type sequencing methods and the like.

Nucleotides labeled with dye compounds of the present disclosure incorporating 3' blocking groups, it will be recognized, may also be of utility in Sanger methods and related protocols since the same effect achieved by using dideoxy nucleotides may be achieved by using nucleotides having 3' OH blocking groups: both prevent incorporation of subsequent nucleotides. Where nucleotides according to the present disclosure, and having a 3' blocking group are to be used in Sanger-type sequencing methods it will be appreciated that the dye compounds or detectable labels attached to the nucleotides need not be connected via cleavable linkers, since in each instance where a labeled nucleotide of the disclosure is incorporated; no nucleotides need to be subsequently incorporated and thus the label need not be removed from the nucleotide.

Alternatively, the sequencing methods described herein may also be carried out using unlabeled nucleotides and affinity reagents containing a fluorescent dye described herein. For example, one, two, three or each of the four different types of nucleotides (e.g., dATP, dCTP, dGTP and dTTP or dUTP) in the incorporation mixture of step (a) may be unlabeled. Each of the four types of nucleotides (e.g., dNTPs) has a 3' hydroxy blocking group to ensure that only a single base can be added by a polymerase to the 3' end of the primer polynucleotide. After incorporation of an unlabeled nucleotide in step (b), the remaining unincorporated nucleotides are washed away. An affinity reagent is then introduced that specifically recognizes and binds to the incorporated dNTP to provide a labeled extension product comprising the incorporated dNTP. Uses of unlabeled nucleotides and affinity reagents in sequencing-by-synthesis have been disclosed in WO 2018/129214 and WO 2020/097607. A modified sequencing method of the present disclosure using unlabeled nucleotides may include the following steps:
(a') contacting a primer polynucleotide/target polynucleotide complex with one or more unlabeled nucleotides (e.g., dATP, dCTP, dGTP, and dTTP or dUTP), wherein the primer polynucleotide is complementary to at least a portion of the target polynucleotide;
(b') incorporating a nucleotide into the primer polynucleotide to produce an extended primer polynucleotide;
(c') contacting the extended primer polynucleotide with a set of affinity reagents under conditions wherein one affinity reagent binds specifically to the incorporated unlabeled nucleotide to provide a labeled extended primer polynucleotide/target polynucleotide complex;
(d') performing one or more fluorescent measurements of the labeled extended primer polynucleotide/target polynucleotide complex to determine the identity of the incorporated nucleotide.

In some embodiments of the modified sequencing method described herein, each of the unlabeled nucleotides in the incorporation mixture contains a 3' hydroxy blocking group. In further embodiments, the 3' hydroxy blocking group of the incorporated nucleotide is removed prior to the next incorporation cycle. In still further embodiments, the method further comprises removing the affinity reagent from the incorporated nucleotide. In still further embodiments, the 3' hydroxy blocking group and the affinity reagent are removed in the same reaction. In some embodiments, the set of affinity reagents may comprise a first affinity reagent that binds specifically to the first type of nucleotide, a second affinity reagent that binds specifically to the second type of nucleotide, and a third affinity reagent that binds specifically to the third type of nucleotide. In some further embodiments, each of the first, second and the third affinity reagents comprises one or more detectable labels that are spectrally distinguishable. In some embodiments, the affinity reagents may include protein tags, antibodies (including but not limited to binding fragments of antibodies, single chain antibodies, bispecific antibodies, and the like), aptamers, knottins, affimers, or any other known agent that binds an incorporated nucleotide with a suitable specificity and affinity. In one embodiment, at least one affinity reagent is an antibody or a protein tag. In another embodiment, at least one of the first type, the second type and the third type of affinity reagents is an antibody or a protein tag comprising one or more detectable labels (e.g., multiple copies of the same detectable label).

### Kits

Further embodiments relates to a kit for chemical linearization of double stranded polynucleotides, comprising a periodate salt and an ionic liquid additive. In some embodiments, the periodate salt and the ionic liquid additive are in an aqueous solution. In other embodiments, at least one or both of the periodate salt and the ionic liquid additive are in a lyophilized form. In such embodiments, the kit may further comprise an aqueous medium such as a buffer solution for reconstituting the periodate salt and the ionic liquid additive.

In some embodiments of the kit described herein, the ionic liquid additive comprises or is selected from a crown ether or a substituted imidazolium salt, or a combination thereof. In some such embodiments, the crown ether comprises or is 15-crown-5. In further embodiments, the molar ratio of the crown ether (e.g. 15-crown-5) to the periodate salt in the composition is about 2:1, 3:1, 4:1, 5:1, 6:1, 7:1, 8:1, 9:1, 10:1, 12:1, 14:1, 16:1, 18:1, 20:1, 25:1 or 30:1. In one embodiment, the molar ratio of the crown ether (e.g. 15-crown-5) to the periodate salt is about 10:1. In other embodiments, the substituted imidazolium salt comprises or is Bzmim]Cl. In some embodiments, the molar ratio of the substituted imidazolium salt (e.g., [Bzmim]Cl) to the periodate salt in the composition is from about 1:1 to about 100:1, from about 5:1 to about 75:1, or from about 10:1 to about 50:1. In further embodiments, the molar ratio of the substituted imidazolium salt (e.g., [Bzmim]Cl) to the periodate salt is about 5:1, 10:1, 15:1, 20:1, 25:1, 30:1, 35:1, 40:1, 45:1 or 50:1, or a range defined by any two of the preceding values. In one embodiment, the molar ratio of the substituted imidazolium salt (e.g., [Bzmim]Cl) to the periodate salt is about 30:1.

In some embodiments, the kit further comprises one or more inorganic salts. For example, the inorganic salt may comprise one or more sodium salts, or one or more magnesium salts, or a combination thereof. In further embodiments, the inorganic salt may comprise sodium citrate, sodium acetate, magnesium sulfate, or combinations thereof.

In further embodiments, the kit may further comprise a second linearization reagent. For example, the second linearization reagent may be an enzyme such as USER for enzymatic cleavage of a cleavage site of a oligonucleotide/polynucleotide strand containing a deoxyuridine (U), such as the P5 primer disclosed herein. In other embodiments, the second linearization may be a chemical cleavage reagent such as a palladium catalyst for cleaving a cleavage site of a oligonucleotide/polynucleotide strand containing a vinyl substituted nucleotide, such as the P15 primer disclosed herein. In such embodiments, the periodate salt/ionic liquid additive is in a different compartment or chamber than the second linearization reagent.

In further embodiments, the kit may further comprise one or more labeled nucleotides and DNA polymerase for SBS as described herein.

In still further embodiments, the kit may further comprise one or more hydroxy protecting reagent to cap the free 3' hydroxy group of the cleaved immobilized first or second strands. The kit may further comprise a 3' hydroxy deprotecting reagent to generate free 3' hydroxy group before polymerase resynthesis or SBS. In some such embodiment, the 3' hydroxy deprotecting reagent may be the same as the Pd catalyst used for chemical cleavage linearization as described herein.

### EXAMPLES

Additional embodiments are disclosed in further detail in the following examples, which are not in any way intended to limit the scope of the claims.

### Example 1. Linearization Percentage Measurements with Various Additives

P17 linearization activity was measured using custom-grafted HiSeq X flow cells (Ilumina), a cBot system (Ilumina) for fluidic and heating operations, and a Typhoon Laser Scanner (Cytiva) for imaging.

The P17 HiSeq X flow cells were grafted with 1.5 µM P17 oligo. Following grafting, the quantity of oligo grafted to the surface of each lane in a P17 flow cell was determined. This pre-linearization quantification was achieved by hybridizing a CFR-modified complementary oligo at 40 °C for 5 minutes, washing away unbound probe, and imaging the entire flow cell with the laser scanner. Following imaging, the complementary oligos were dehybridized from the surface by flushing the surface with 0.1 M NaOH for 5 minutes.

The diol cleavage rate was measured using a real-time FRET-based assay on a Cytation fluorescence plate reader (BioTek Instruments). The assay used a modified oligo (Integrated DNA Technologies) containing an ATTO 610 fluorophore in close proximity to a BHQ-2 quenching moiety, with a diol cleavage site separating the two modifications. Before linearization, BHQ-2 quenches ATTO 610 fluorescence. Linearization starts after the sodium periodate solution is added to the samples, and fluorescence measurements begins immediately thereafter. The oligo splits and the quencher diffuses away from ATTO 610. Real-time fluorescence monitoring can be used to follow the rate of linearization.

A 1.6 mM sodium periodate solution was prepared by dissolving sodium periodate (Sigma-Aldrich) in pure water. In some samples, either 10 equivalents of 15-crown-5 (Sigma-Aldrich) or 30.4 equivalents of [Bzmim]Cl (Sigma-Aldrich) was further added to the sodium periodate solution. To measure the linearization activities for a set of samples containing sodium periodate, each sample was pumped through one lane of a P17-grafted flow cell. The flow cell was incubated at 20 °C for 10 minutes or the appropriate duration. The quantity of oligo remaining on the surface of each lane post-linearization was measured using the same approach as the pre-linearization quantification. Percentage linearization was determined by dividing each lane's post-linearization value by its pre-linearization value.

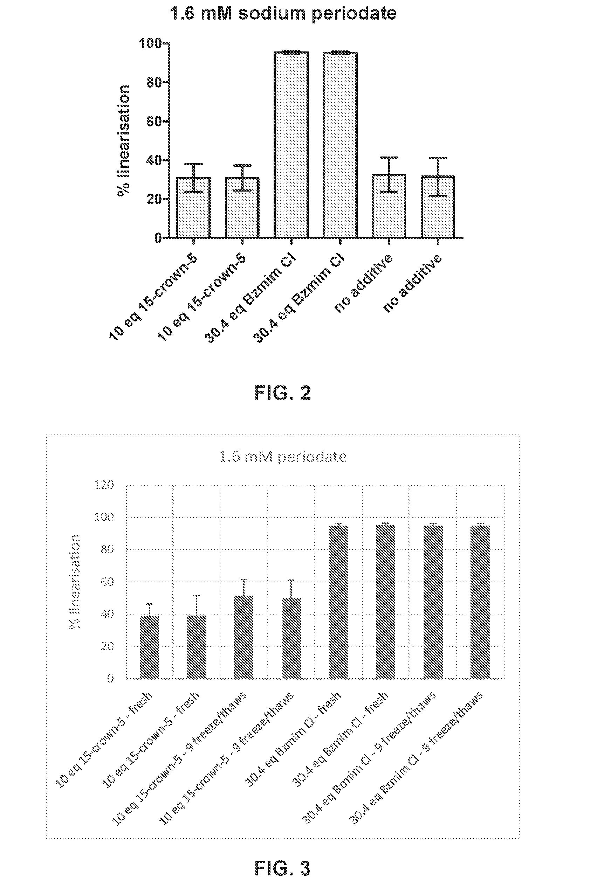

It has been shown that the linearization activity improved with the addition of different ionic liquid additives to the sodium periodate solution. **FIG. 2** is a bar chart of percent linearization of double-stranded polynucleotides in a composition comprising sodium periodate when different ionic liquid additive is added. As illustrated in **FIG. 2**, the addition of about 30 molar equivalents of [Bzmim]Cl increased the periodate linearization activity threefold. **FIG. 3** illustrates the percent linearization of double-stranded polynucleotides in a composition comprising 1.6 mM sodium periodate either freshly prepared or has been subject to freeze/thaw cycles when either 10 equivalents of 15-crown-5 or 30.4 equivalents of [Bzmim]Cl was further added to the sodium periodate solution. It was observed that 15-crown-5 improved the percent linearization of the sodium periodate solution that was subject to 9 freeze/thaw cycles as compared to the freshly prepared control. [Bzmim]Cl substantially improved the percent linearization in both fresh prepared sodium periodate solution and sodium periodate solution that was subject to 9 freeze/thaw cycles. The linearization activity of sodium periodate solution after 9 freeze/thaws cycles was equivalent to the linearization activities observed in the freshly prepared sodium periodate when 30.4 eq of [Bzmim]Cl was added.

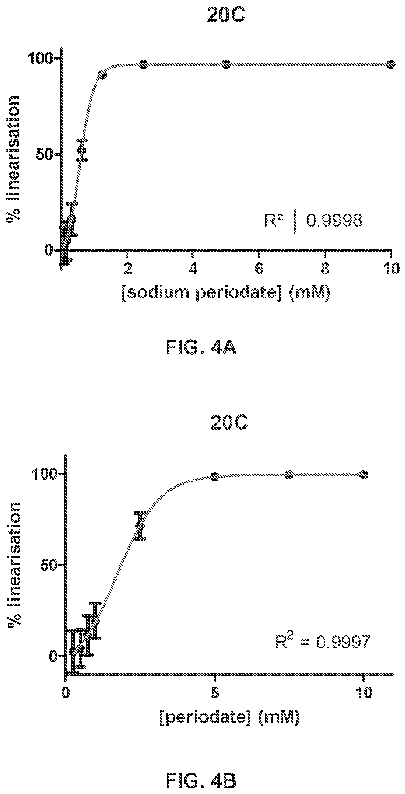

Furthermore, as illustrated in **FIGs. 4A** and **4B****,** the V50 of sodium periodate concentration was 1.72 mM without [Bzmim]Cl **(****FIG. 4B****)** and the V50 of sodium periodate concentration was 0.56 mM with 30.4 molar equivalent of [Bzmim]Cl added **(****FIG. 4A****).** It has been shown that the addition of about 30 molar equivalents of [Bzmim]Cl increased the periodate linearization activity threefold at 20 °C.

### Example 2. Measurement of Initial Diol Cleavage Rates of Fresh and Aged Sodium Periodate

The initial diol cleavage rate (*v*₀) was measured using a real-time FRET-based assay on a Cytation fluorescence plate reader (BioTek Instruments). The assay used a modified oligo (Integrated DNA Technologies) containing an ATTO 610 fluorophore in close proximity to a BHQ-2 quenching moiety, with a diol cleavage site separating the two modifications. Before linearization, BHQ-2 quenches ATTO 610 fluorescence. Linearization starts after the sodium periodate solution is added to the samples, and fluorescence measurements begins immediately thereafter. The oligo splits and the quencher diffuses away from ATTO 610. Real-time fluorescence monitoring can be used to follow the rate of linearization.

In this experiment, sodium periodate solution containing 30.4 equivalent of [Bzmim]Cl was prepared according to the description in Example 1. An autoinjector was used to deliver the sample of sodium periodate into a solution of the FRET oligo in a well of a 96-well plate, which initiated the linearization reaction. The progress was tracked by monitoring the fluorescence in the ATTO 610 channel. As the oligo was linearized, the fluorescent dye and quencher diffused away from each other, causing the fluorescence signal to increase.

Initial rate of the reaction was determined by performing a linear regression of the first few points of the fluorescence versus time plot and measuring the slope. For precise determination of the initial rate of reaction, samples were diluted to 1 mM sodium periodate to slow the reaction.

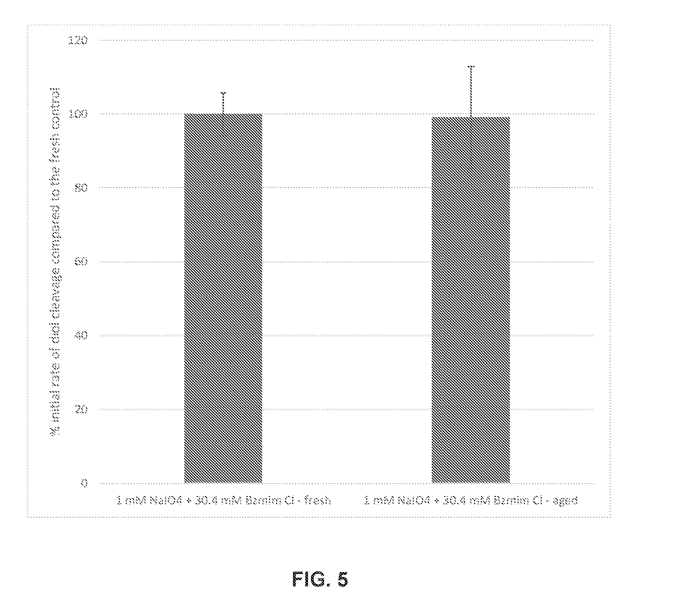

**FIG. 5** is a bar chart comparing the percent of initial rate of diol cleavage of double-stranded polynucleotides using a freshly prepared sodium periodate + 30.4 equivalent of [Bzmim]Cl composition compared to the same composition that was aged in air at 60°C for 10 days. It was observed that the sodium periodate composition containing the [Bzmim]Cl additive was very stable and no meaningful change in the initial rate of diol cleavage was observed in the aged sodium periodate composition.

## Claims

1. A composition comprising a periodate salt and an ionic liquid additive, wherein the ionic liquid additive is a substituted imidazolium salt, wherein the periodate is dissolved in an aqueous solution, and the periodate salt does not form a precipitate in the aqueous solution.

2. The composition of claim 1, wherein:
(i) the periodate salt comprises sodium periodate; and/or
(ii) the concentration of the periodate salt in the composition is from 0.1 mM to 300 mM, from 0.5 mM to 200 mM, from 1 mM to 150 mM, from 2 mM to 100 mM, or from 5 mM to 50 mM, optionally
wherein the concentration of the periodate salt in the composition is 10 mM or 25 mM.

3. The composition of claim 1 or claim 2, wherein the substituted imidazolium salt comprises 1-benzyl-3-methylimidazolium chloride ([Bzmim]Cl).

4. The composition of claim 3, wherein the molar ratio of [Bzmim]Cl to the periodate salt in the composition is from 1:1 to 100:1, or from 10:1 to 50:1; optionally
wherein the molar ratio of [Bzmim]Cl to the periodate salt in the composition is about 30:1.

5. The composition of any one of claims 1 to 4, wherein :
(i) the composition further comprises one or more inorganic salts; optionally
wherein the inorganic salt comprises sodium citrate, sodium acetate, or magnesium sulfate, or combinations thereof;and/or
(ii) the pH of the composition is from 4 to 8, or from 5 to 7.5; optionally
wherein the pH of the composition is 6; and/or
(iii) no precipitate of the periodate salt is formed after the composition is subject to at least 5 freeze/thaw cycles.

6. A method of linearizing a plurality of immobilized double-stranded polynucleotides, comprising:
contacting a periodate salt composition of any one of claims 1 to 5 with a solid support comprising the plurality of immobilized double-stranded polynucleotides, wherein each double-stranded polynucleotide comprises a first strand and a second strand, wherein the first strand and the second strand are immobilized to the solid support at their 5' ends, wherein each second strand comprises a cleavage site; and
chemically cleaving one or more second strands at the cleavage site with the periodate salt and generating one or more cleaved second nucleic acids.

7. The method of claim 6, wherein (i) the method further comprises removing the cleaved second nucleic acids from the solid support; and/or
(ii) the cleavage site of each second strand comprises one or more diol linkers, optionally
wherein the diol linker comprises a structure of Formula (I): 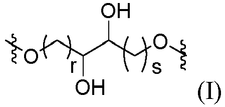 wherein
r is 2, 3, 4, 5, or 6; and
s is 2, 3, 4, 5, or 6
or a structure of Formula (Ia): 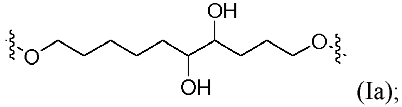 and/or
(iii) the second strand polynucleotide comprises a P17 sequence.

8. A method of sequencing polynucleotides, comprising:
contacting a palladium catalyst with a solid support comprising a plurality of immobilized double-stranded polynucleotides, wherein each double-stranded polynucleotide comprises a first strand and a second strand, wherein the first strand and the second strand are immobilized to the solid support at their 5' ends, wherein each first strand comprises a first cleavage site comprising a vinyl moiety, and wherein each second strand comprises a second cleavage site comprising one or more diol linkers;
cleaving one or more first strands at the first cleavage site with the palladium catalyst, and generating one or more cleaved first nucleic acids and cleaved immobilized first strands;
sequencing the immobilized second strands;
resynthesizing derivative first strands that are complementary to the second strands;
contacting a periodate salt composition of any one of claims 1 to 5 with one or more second strands to cleave the second strands at the second cleavage site, and generating one or more cleaved second nucleic acids and cleaved immobilized second strands; and
sequencing the immobilized derivative first strands.

9. The method of claim 8, wherein (i) the first cleavage site comprises a modified nucleotide comprising a structure of Formula (II): 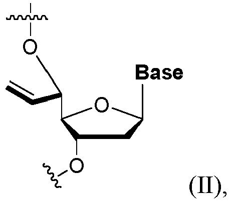
wherein Base is adenine, 7-deazaademine, guanine, 7-deazaguanine, cytosine, thymine, or uracil, or a derivative thereof; and/or
(ii) each first strand is extended from a first extension primer immobilized to the solid support, and wherein the first extension primer comprises a P15 sequence.

10. The method of claim 8 or claim 9, wherein (i) the diol linker comprises a structure of Formula (I): 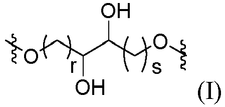 wherein
r is 2, 3, 4, 5, or 6; and
s is 2, 3, 4, 5, or 6;
optionally wherein the structure of Formula (I) is a structure of Formula (Ia): 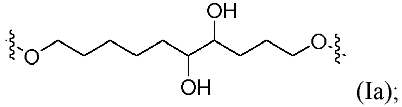 and/or
(ii) each second strand is extended from a second extension primer immobilized to the solid support, and wherein the second extension primer comprises a P17 sequence.

11. A kit for chemical linearization of double stranded polynucleotides, comprising a periodate salt and an ionic liquid additive wherein the ionic liquid additive is a substituted imidazolium salt, wherein at least one of the periodate salt and the ionic liquid additive is in a lyophilized form.

12. The kit of claim 11, wherein the periodate salt comprises sodium periodate.

13. The kit of claim 11 or claim 12, wherein the substituted imidazolium salt comprises 1-benzyl-3-methylimidazolium chloride ([Bzmim]Cl).

14. The kit of claim 13, wherein the molar ratio of [Bzmim]Cl to the periodate salt is from 1:1 to 100:1, from 10:1 to 50:1, or 30:1.

15. The kit of any one of claims 11 to 14, further comprising one or more inorganic salts, optionally
wherein the inorganic salt comprises sodium citrate, sodium acetate, or magnesium sulfate, or combinations thereof.

## Patentansprüche

1. Eine Zusammensetzung, die ein Periodatsalz und ein ionisches Flüssigadditiv beinhaltet, wobei das ionische Flüssigadditiv ein substituiertes Imidazoliumsalz ist, wobei das Periodat in einer wässrigen Lösung gelöst ist und das Periodatsalz in der wässrigen Lösung kein Präzipitat bildet.

2. Zusammensetzung gemäß Anspruch 1, wobei:
(i) das Periodatsalz Natriumperiodat beinhaltet; und/oder
(ii) die Konzentration des Periodatsalzes in der Zusammensetzung 0,1 mM bis 300 mM, 0,5 mM bis 200 mM, 1 mM bis 150 mM, 2 mM bis 100 mM oder 5 mM bis 50 mM beträgt,
wobei die Konzentration des Periodatsalzes in der Zusammensetzung optional 10 mM oder 25 mM beträgt.

3. Zusammensetzung gemäß Anspruch 1 oder Anspruch 2, wobei das substituierte Imidazoliumsalz 1-Benzyl-3-methylimidazoliumchlorid ([Bzmim]Cl) beinhaltet.

4. Zusammensetzung gemäß Anspruch 3, wobei das Molverhältnis von [Bzmim]CI zu dem Periodatsalz in der Zusammensetzung 1 : 1 bis 100 : 1 oder 10 : 1 bis 50 : 1 beträgt; wobei das Molverhältnis von [Bzmim]CI zu dem Periodatsalz in der Zusammensetzung optional ungefähr 30 : 1 beträgt.

5. Zusammensetzung gemäß einem der Ansprüche 1 bis 4, wobei:
(i) die Zusammensetzung ferner ein oder mehrere anorganische Salze beinhaltet;
wobei das anorganische Salz optional Natriumcitrat, Natriumacetat oder Magnesiumsulfat oder Kombinationen davon beinhaltet; und/oder
(ii) der pH-Wert der Zusammensetzung 4 bis 8 oder 5 bis 7,5 beträgt;
wobei der pH-Wert der Zusammensetzung optional 6 beträgt; und/oder
(iii) kein Präzipitat des Periodatsalzes gebildet wird, nachdem die Zusammensetzung mindestens 5 Frost-Tau-Zyklen ausgesetzt war.

6. Ein Verfahren zum Linearisieren einer Vielzahl immobilisierter doppelsträngiger Polynukleotide, beinhaltend:
In-Kontakt-Bringen einer Periodatsalzzusammensetzung gemäß einem der Ansprüche 1 bis 5 mit einem festen Träger, der eine Vielzahl immobilisierter doppelsträngiger Polynukleotide beinhaltet, wobei jedes doppelsträngige Polynukleotid einen ersten Strang und einen zweiten Strang beinhaltet, wobei der erste Strang und der zweite Strang an ihrem 5'-Ende an dem festen Träger immobilisiert sind, wobei jeder zweite Strang eine Spaltstelle beinhaltet; und
chemisches Spalten eines oder mehrerer zweiter Stränge an der Spaltstelle mit dem Periodatsalz und Erzeugen einer oder mehrerer abgespaltener zweiter Nukleinsäuren.

7. Verfahren gemäß Anspruch 6, wobei (i) das Verfahren ferner das Entfernen der abgespaltenen zweiten Nukleinsäuren von dem festen Träger beinhaltet; und/oder
(ii) die Spaltstelle jedes zweiten Strangs einen oder mehrere Diollinker beinhaltet,
wobei der Diollinker optional eine Struktur der Formel (I) beinhaltet: 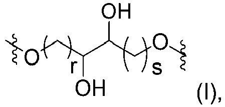
wobei
r 2, 3, 4, 5 oder 6 ist; und
s 2, 3, 4, 5 oder 6 ist;
oder eine Struktur der Formel (la): 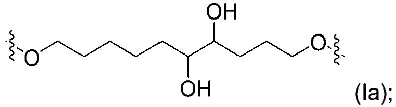 und/oder
(iii) das Polynukleotid des zweiten Strangs eine P17-Sequenz beinhaltet.

8. Ein Verfahren zum Sequenzieren von Polynukleotiden, beinhaltend:
In-Kontakt-Bringen eines Palladiumkatalysators mit einem festen Träger, der eine Vielzahl immobilisierter doppelsträngiger Polynukleotide beinhaltet, wobei jedes doppelsträngige Polynukleotid einen ersten Strang und einen zweiten Strang beinhaltet,
wobei der erste Strang und der zweite Strang an ihrem 5'-Ende an dem festen Träger immobilisiert sind, wobei jeder erste Strang eine erste Spaltstelle beinhaltet, die einen Vinylteil beinhaltet, und wobei jeder zweite Strang eine zweite Spaltstelle beinhaltet, die einen oder mehrere Diollinker beinhaltet;
Spalten eines oder mehrerer erster Stränge an der ersten Spaltstelle mit dem Palladiumkatalysator und Erzeugen einer oder mehrerer abgespaltener erster Nukleinsäuren und eines oder mehrerer abgespaltener immobilisierter erster Stränge;
Sequenzieren der immobilisierten zweiten Stränge;
Resynthetisieren derivativer erster Stränge, die zu den zweiten Strängen komplementär sind;
In-Kontakt-Bringen einer Periodatsalzzusammensetzung gemäß einem der Ansprüche 1 bis 5 mit einem oder mehreren zweiten Strängen, um die zweiten Stränge an der zweiten Spaltstelle zu spalten, und Erzeugen einer oder mehrerer abgespaltener zweiter Nukleinsäuren und abgespaltener immobilisierter zweiter Stränge; und
Sequenzieren der immobilisierten derivativen ersten Stränge.

9. Verfahren gemäß Anspruch 8, wobei (i) die erste Spaltstelle ein modifiziertes Nukleotid beinhaltet, das eine Struktur der Formel (II) beinhaltet: 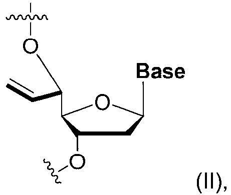
wobei die Base Adenin, 7-Desazaadenin, Guanin, 7-Desazaguanin, Cytosin, Thymin oder Uracil oder ein Derivat davon ist; und/oder
(ii) jeder erste Strang ab einem ersten an dem festen Träger immobilisierten Verlängerungsprimer verlängert wird und wobei der erste Verlängerungsprimer eine P15-Sequenz beinhaltet.

10. Verfahren gemäß Anspruch 8 oder Anspruch 9, wobei (i) der Diollinker eine Struktur der Formel (I) beinhaltet: 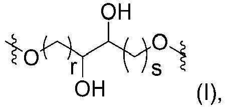 wobei
r 2, 3, 4, 5 oder 6 ist; und
s 2, 3, 4, 5 oder 6 ist;
wobei die Struktur der Formel (I) optional eine Struktur der Formel (la) ist: 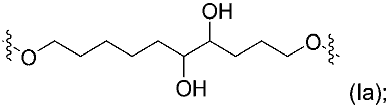 und/oder
(ii) jeder zweite Strang ab einem zweiten an dem festen Träger immobilisierten Verlängerungsprimer verlängert wird und wobei der zweite Verlängerungsprimer eine P17-Sequenz beinhaltet.

11. Ein Kit zur chemischen Linearisierung doppelsträngiger Polynukleotide, das ein Periodatsalz und ein ionisches Flüssigadditiv beinhaltet, wobei das ionische Flüssigadditiv ein substituiertes Imidazoliumsalz ist, wobei mindestens eines von dem Periodatsalz und dem ionischen Flüssigadditiv in gefriergetrockneter Form vorliegt.

12. Kit gemäß Anspruch 11, wobei das Periodatsalz Natriumperiodat beinhaltet.

13. Kit gemäß Anspruch 11 oder Anspruch 12, wobei das substituierte Imidazoliumsalz 1-Benzyl-3-methylimidazoliumchlorid ([Bzmim]Cl) beinhaltet.

14. Kit gemäß Anspruch 13, wobei das Molverhältnis von [Bzmim]CI zu dem Periodatsalz 1 : 1 bis 100 : 1, 10 : 1 bis 50 : 1 oder 30 : 1 beträgt.

15. Kit gemäß einem der Ansprüche 11 bis 14, das ferner ein oder mehrere anorganische Salze beinhaltet,
wobei das anorganische Salz optional Natriumcitrat, Natriumacetat oder Magnesiumsulfat oder Kombinationen davon beinhaltet.

## Revendications

1. Une composition comprenant un sel de périodate et un additif liquide ionique, où l'additif liquide ionique est un sel d'imidazolium substitué, où le périodate est dissous dans une solution aqueuse, et le sel de périodate ne forme pas de précipité dans la solution aqueuse.

2. La composition de la revendication 1, où :
(i) le sel de périodate comprend du périodate de sodium ; et/ou
(ii) la concentration du sel de périodate dans la composition est de 0,1 mM à 300 mM, de 0,5 mM à 200 mM, de 1 mM à 150 mM, de 2 mM à 100 mM, ou de 5 mM à 50 mM, facultativement
où la concentration du sel de périodate dans la composition est de 10 mM ou de 25 mM.

3. La composition de la revendication 1 ou de la revendication 2, où le sel d'imidazolium substitué comprend du chlorure de 1-benzyl-3-méthylimidazolium ([Bzmim]Cl).

4. La composition de la revendication 3, où le rapport molaire de [Bzmim]Cl au sel de périodate dans la composition est de 1/1 à 100/1, ou de 10/1 à 50/1 ; facultativement où le rapport molaire de [Bzmim]Cl au sel de périodate dans la composition est d'environ 30/1.

5. La composition de l'une quelconque des revendications 1 à 4, où :
(i) la composition comprend en outre un ou plusieurs sels inorganiques ; facultativement
où le sel inorganique comprend du citrate de sodium, de l'acétate de sodium, ou du sulfate de magnésium, ou des combinaisons de ceux-ci ; et/ou
(ii) le pH de la composition est de 4 à 8, ou de 5 à 7,5 ; facultativement
où le pH de la composition est de 6 ; et/ou
(iii) aucun précipité du sel de périodate n'est formé après que la composition a été soumise à au moins 5 cycles de congélation/décongélation.

6. Un procédé de linéarisation d'une pluralité de polynucléotides double brin immobilisés, comprenant :
la mise en contact d'une composition de sel de périodate de l'une quelconque des revendications 1 à 5 avec un support solide comprenant la pluralité de polynucléotides double brin immobilisés, où chaque polynucléotide double brin comprend un premier brin et un deuxième brin, où le premier brin et le deuxième brin sont immobilisés sur le support solide au niveau de leurs extrémités en 5', où chaque deuxième brin comprend un site de clivage ; et
le clivage chimique d'un ou de plusieurs deuxièmes brins au niveau du site de clivage avec le sel de périodate et la génération d'un ou de plusieurs deuxièmes acides nucléiques clivés.

7. Le procédé de la revendication 6, où (i) le procédé comprend en outre l'élimination des deuxièmes acides nucléiques clivés du support solide ; et/ou
(ii) le site de clivage de chaque deuxième brin comprend un ou plusieurs lieurs diol, facultativement
où le lieur diol comprend une structure de Formule (I) : 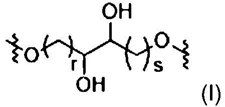
où
r vaut 2, 3, 4, 5 ou 6 ; et
s vaut 2, 3, 4, 5 ou 6
ou une structure de Formule (la) : 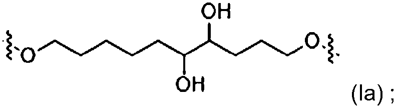 et/ou
(iii) le polynucléotide de deuxième brin comprend une séquence P17.

8. Un procédé de séquençage de polynucléotides, comprenant :
la mise en contact d'un catalyseur au palladium avec un support solide comprenant une pluralité de polynucléotides double brin immobilisés, où chaque polynucléotide double brin comprend un premier brin et un deuxième brin, où le premier brin et le deuxième brin sont immobilisés sur le support solide au niveau de leurs extrémités en 5', où chaque premier brin comprend un premier site de clivage comprenant un groupement vinyle, et où chaque deuxième brin comprend un deuxième site de clivage comprenant un ou plusieurs lieurs diol ;
le clivage d'un ou de plusieurs premiers brins au niveau du premier site de clivage avec le catalyseur au palladium, et la génération d'un ou de plusieurs premiers acides nucléiques clivés et de premiers brins immobilisés clivés ;
le séquençage des deuxièmes brins immobilisés ;
la resynthèse de premiers brins dérivés qui sont complémentaires des deuxièmes brins ;
la mise en contact d'une composition de sel de périodate de l'une quelconque des revendications 1 à 5 avec un ou plusieurs deuxièmes brins pour cliver les deuxièmes brins au niveau du deuxième site de clivage, et la génération d'un ou de plusieurs deuxièmes acides nucléiques clivés et de deuxièmes brins immobilisés clivés ; et
le séquençage des premiers brins dérivés immobilisés.

9. Le procédé de la revendication 8, où (i) le premier site de clivage comprend un nucléotide modifié comprenant une structure de Formule (II) : 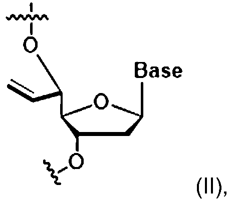
où Base est l'adénine, la 7-déazaadémine, la guanine, la 7-déazaguanine, la cytosine, la thymine, ou l'uracile, ou un dérivé de ceux-ci ; et/ou
(ii) chaque premier brin est étendu à partir d'une première amorce d'extension immobilisée sur le support solide, et où la première amorce d'extension comprend une séquence P15.

10. Le procédé de la revendication 8 ou de la revendication 9, où (i) le lieur diol comprend une structure de Formule (1) : 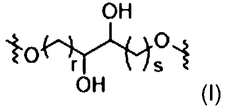 où
r vaut 2, 3, 4, 5 ou 6 ; et
s vaut 2, 3, 4, 5 ou 6 ;
facultativement où la structure de Formule (I) est une structure de Formule (la) : 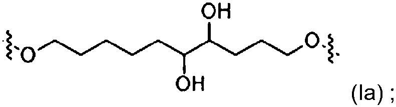 et/ou
(ii) chaque deuxième brin est étendu à partir d'une deuxième amorce d'extension immobilisée sur le support solide, et où la deuxième amorce d'extension comprend une séquence P17.

11. Un kit pour la linéarisation chimique de polynucléotides double brin, comprenant un sel de périodate et un additif liquide ionique où l'additif liquide ionique est un sel d'imidazolium substitué, où au moins l'un du sel de périodate et de l'additif liquide ionique est sous une forme lyophilisée.

12. Le kit de la revendication 11, où le sel de périodate comprend du périodate de sodium.

13. Le kit de la revendication 11 ou de la revendication 12, où le sel d'imidazolium substitué comprend du chlorure de 1-benzyl-3-méthylimidazolium ([Bzmim]Cl).

14. Le kit de la revendication 13, où le rapport molaire de [Bzmim]Cl au sel de périodate est de 1/1 à 100/1, de 10/1 à 50/1, ou de 30/1.

15. Le kit de l'une quelconque des revendications 11 à 14, comprenant en outre un ou plusieurs sels inorganiques, facultativement
où le sel inorganique comprend du citrate de sodium, de l'acétate de sodium, ou du sulfate de magnésium, ou des combinaisons de ceux-ci.
